(19) Europäisches Patentamt
European Patent Office
Office européen des brevets

(11) Veröffentlichungsnummer : **0 234 348 B1**

(12) # EUROPÄISCHE PATENTSCHRIFT

(45) Veröffentlichungstag der Patentschrift :
**29.05.91 Patentblatt 91/22**

(51) Int. Cl.$^5$ : **C07H 15/12**, C07H 15/04, A61K 31/70, // C07H13/04

(21) Anmeldenummer : **87101462.7**

(22) Anmeldetag : **03.02.87**

(54) **Neue N-Glycosylamidderivate, Verfahren zu ihrer Herstellung sowie ihre Verwendung als Arzneimittel.**

(30) Priorität : **14.02.86 DE 3604681**

(43) Veröffentlichungstag der Anmeldung :
**02.09.87 Patentblatt 87/36**

(45) Bekanntmachung des Hinweises auf die Patenterteilung :
**29.05.91 Patentblatt 91/22**

(84) Benannte Vertragsstaaten :
**AT BE CH DE ES FR GB IT LI NL SE**

(56) Entgegenhaltungen :
**EP-A- 0 091 645**
**EP-A- 0 100 104**

(73) Patentinhaber : **BAYER AG**
**W-5090 Leverkusen 1 Bayerwerk (DE)**

(72) Erfinder : **Lockhoff, Oswald, Dr.**
**Morgengraben 14**
**W-5000 Köln 80 (DE)**
Erfinder : **Hayauchi, Yutaka, Dr.**
**Gustav-Freytag-Strasse 4**
**W-5090 Leverkusen 1 (DE)**
Erfinder : **Stadler, Peter, Dr.**
**Am Ideck 8**
**W-5657 Haan 1 (DE)**
Erfinder : **Brunner, Helmut, Prof.Dr.**
**Waldstrasse 10**
**W-4018 Langenfeld (DE)**

**EP 0 234 348 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue N-Glycosylamidderivate, Verfahren zu ihrer Herstellung sowie ihre Verwendung als Arzneimittel.

Die neuen Verbindungen entsprechen der allgemeinen Formel I

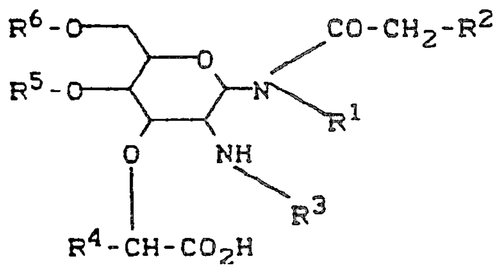

(I)

Hierin bedeutet

$R^1$ Wasserstoff oder einen gesättigten oder einfach oder mehrfach ungesättigten Alkylrest mit einem bis zu 50 Kohlenstoffatomen.

$R^2$ Wasserstoff oder einen gesättigten oder einfach oder mehrfach ungesättigten Alkylrest mit einem bis zu 50 Kohlenstoffatomen,

$R^3$ Wasserstoff oder einen Acylrest mit bis zu 20 Kohlenstoffatomen,

$R^4$ Wasserstoff oder einen Alkylrest mit bis zu 4 Kohlenstoffatomen,

$R^5$ und $R^6$ unabhängig voneinander Wasserstoff oder einen Acylrest mit bis zu 10 Kohlenstoffatomen oder einen gegebenenfalls ungesättigten Alkylrest oder einen Aralkylrest mit bis zu 10 Kohlenstoffatomen,

$R^5$ und $R^6$ zusammen eine Gruppierung

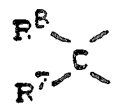

wobei $R^7$ und $R^8$ unabhängig voneinander Wasserstoff oder Niederalkyl mit bis zu 5 Kohlenstoffatomen oder einen gegebenenfalls substituierten Arylrest bedeuten.

Der Rest $R^1$ stellt bevorzugt einen geradkettigen oder verzweigten, gesättigten oder einfach oder mehrfach ungesättigter, Alkylrest mit bis zu 22 Kohlenstoffatomen dar. Besonders bevorzugt für $R^1$ sind geradkettige, gesättigte oder einfach ungesättigte Alkylreste mit 10 bis 20 Kohlenstoffatomen.

Beispiele für geradkettige, gesättigte Alkylreste $R^1$ sind Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Heneicosyl, Docosyl, Tricosyl, Tetracosyl, Hexacosyl, Octacosyl, Triacontyl.

Beispiele für ungesättigte Alkylreste sind Vinyl, Allyl, But-2-enyl, Hex-3-enyl, Hex-2-enyl, Hex-3-enyl, Hex-4-enyl, Hex-5-enyl, Oct-2-enyl, Oct-4-enyl, Oct-6-enyl, Dec-2-enyl, Dec-4-enyl, Dec-6-enyl, Dec-8-enyl, Dodec-2-enyl, Dodec-4-enyl, Dodec-6-enyl, Dodec-8-enyl, Dodec-10-enyl, Tetradec-2-enyl, Tetradec-4-enyl, Tetradec-6-enyl, Tetradec-8-enyl, Tetradec-10-enyl, Tetradec-12-enyl, Hexadec-2-enyl, Hexadec-4-enyl, Hexadec-6-enyl, Hexadec-8-enyl, Hexadec-10-enyl, Hexadec-12-enyl, Hexadec-14-enyl, Octadec-2-enyl, Octadec-4-enyl, Octadec-6-enyl, Octadec-8-enyl, Octadec-10-enyl, Octadec-12-enyl, Octadec-14-enyl, Octadec-16-enyl, Heptadec-8,11-dienyl, Heptadec-8,11,14-trienyl.

Der Rest $R^2$ stellt bevorzugt einen geradkettigen oder verzweigten, gesättigten oder einfach oder zweifach ungesättigten Alkylrest mit bis zu 22 Kohlenstoffatomen dar, besonders bevorzugt für $R^2$ sind geradkettige, gesättigte oder einfach ungesättigte Alkylreste mit 10 bis 20 Kohlenstoffatomen.

Beispiele für die Rest $R^2$ sind die unter $R^1$ genannten.

Der Rest $R^3$ stellt bevorzugt Wasserstoff oder einen kurskettigen Acylrest mit bis zu 5 Kohlenstoffatomen dar ; besonders bevorzugt sind kurzkettige Acylreste.

Beispiele für $R^3$ sind Formyl, Acetyl, Propionyl, Butyryl oder Valeryl.

Beispiele für die Alkylreste $R^4$ sind Methyl, Ethyl, Propyl, Butyl.

Der Rest $R^5$ stellt bevorzugt Wasserstoff oder einen kurzettigen, gesättigten Acylsubstituenten wie Acetyl oder Propionyl oder einen aromatischen Acylsubstituenten wie Benzoyl oder p-Methoxybenzoyl oder einen Alkylrest wie Allyl oder Benzyl oder p-Methoxybenzyl dar. Besonders bevorzugt in der Bedeutung für $R^5$ ist Was-

serstoff.

Für den Rest $R^6$ gilt das bei dem Rest $R^5$ Gesagte.

Bevorzugte Beispiele für solche Gruppen, in denen $R^5$ und $R^6$ zusammen eine Gruppierung

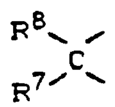

mit der oben angegebenen Bedeutung von $R^7$ und $R^8$ bilden, sind Benzyliden, p-Methoxybenzyliden oder Isopropyliden.

Wie in Formel I ersichtlich ist, liegt den erfindungsgemäßen Verbindungen eine substituierte 2-Amino-2-desoxyhexose zugrunde. Diese Aminozucker sind immer über C-1, das anomere Kohlenstoffatom, N-glycosidisch mit der Acylamidogruppe

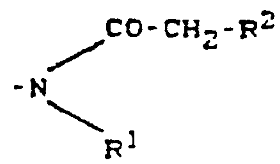

mit den oben angegebenen Bedeutungen für $R^1$ und $R^2$ verbunden.

Bevorzugte Aminozucker in den erfindungsgemäßen Verbindungen der Formel I sind 2-Amino-2-desoxy-D-glucose und 2-Amino-2-desoxy-D-galactose.

Wie in der allgemeinen Formel weiter zu ersehen ist, ist die C-3-Hydroxylgruppe des Aminozuckers mit einer alpha-Hydroxycarbonsäure verethert. Die Stereochemie an dem alpha-Kohlenstoffatom der Hydroxycarbonsäure ist R oder S, bevorzugt jedoch ist die R-Konfiguation.

Die Erfindung betrifft auch Verfahren für die Herstellung der erfindungsgemäßen Verbindungen der allgemeinen Formel I.

Hierbei setzt man die an der Aminogrupe acylierten Derivate der 2-Amino-2-desoxy-hexose der allgemeinen Formel II

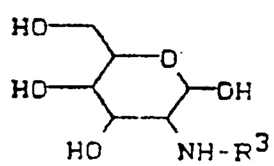

II

mit der oben genannten Bedeutung von $R^3$ um mit Aminoverbindungen $R^1$-$NH_2$ mit der oben genannten Bedeutung für $R^1$ zu einem Glycosylamin der allgemeinen Formel III

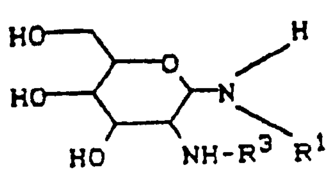

III

mit den oben genannten Bedeutungen für $R^1$ und $R^3$. Das Glycosylamin der allgemeinen Formel III wird dann nach dessen Isolierung mit einem – wie bei Acylierungsreaktionen üblich – aktivierten Carbonsäurederivat selektiv N-acyliert zum Glycosylamid der allgemeinen Formel IV,

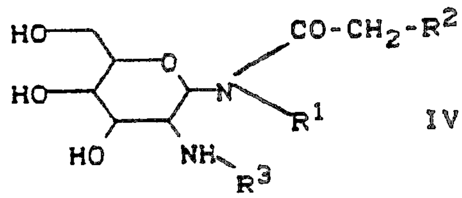

IV

mit den oben angegebenen Bedeutungen von $R^1$, $R^2$ und $R^3$.

Die Herstellung der Verbindungen der allgemeinen Formel IV ist bekannt und ist z.B. in der DE-OS 3 213 650 beschrieben.

In dem nächsten Reaktionsschritt worden die Verbindungen der allgemeinen Formel IV mit einem Aldehyd oder einem Keton oder einem Aldehydderivat oder einem Ketonderivat zu den Verbindungen der allgemeinen Formel V umgesetzt.

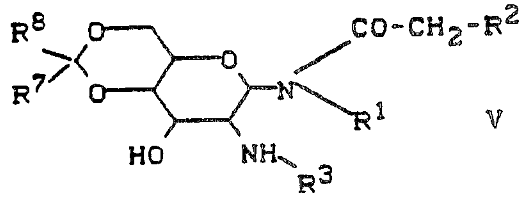

V

Geeignete Aldehyde sind beispielsweise Acetaldehyd, Propionaldehyd, Butyraldehyd, Valeraldehyd, Benzaldehyd oder p-Methoxybenzaldehyd.

Geeignete Ketone sind beispielsweise Aceton, Methylethylketon, Methylpropylketon, Methylbutylketon, Methylpentylketon, Diethylketon, Ethylpropylketon oder Dipropylketon.

Geeignete Aldehydderivate sind solche bereits aktivierten Derivate, die in Umacetalisierungsreaktionen unter Bildung relativ leicht zu entfernender Spaltprodukte mit Diolen zu 1,3-Dioxalanringen oder 1,3-Dioxanringen reagieren. Geeignete Aldehydderivate sind also z.B. Acetale wie Acetaldehyddimethylacetal, Acetaldehyddiethylacetal, Propionaldehyddimethylacetal, Propionaldehyddiethylacetal oder aromatische Acetalderivate wie Benzaldehyddimethylacetal, Benzaldehyddiethylacetal oder gegebenenfalls im Aromaten substituierte Acetale wie p-Tolualdehyddimethylacetal, p-Tolualdehyddiethylacetal oder p-Anisaldehyddimethylacetal oder p-Anisaldehyddiethylacetal. Geeignete Ketonderivate sind beispielsweise Ketale wie 2,2-Dimethoxypropan, 2,2-Diethoxypropan, 2,2-Dimethoxybutan, 2,2-Diethoxybutan, 2,2-Dimethoxypentan, 2,2-Diethoxypentan, 3,3-Dimethoxypentan, Acetophenondimethylketal, Acetophenondiethylketal. Geeignete Ketonderivate für Ketalisierungen sind ferner Vinylether wie z.B. 2-Propen-2-yl-methylether.

Die Aldehyde oder Ketone oder Aldehydderivate oder Ketonderivate oder Vinylether werden mit den Triolen der allgemeinen Formel IV in einem geeigneten Lösungsmittel umgesetzt. Geeignete Lösungsmittel sind inerte organische Lösungsmittel wie beispielsweise Tetrahydrofuran, 1,4-Dioxan, Dimethylformamid, Dichlormethan, Chloroform, Ethylenglycoldimethylether, entweder als reine Lösungsmittel oder als Mischungen untereinander.

Die Triole der allgemeinen Formel IV können auch in einem Überschuß des als Lösungsmittel und Reagenz fungierenden Aldehyds oder Ketons oder Aldehydderivats oder Ketonderivats zu den Dioxolanen der allgemeinen Formel V umgesetzt werden. Diese Verfahrensvariante ist z.B. bei der Herstellung von 1,3-Dioxanen der allgemeinen Formel V, in der sowohl $R^7$ als auch $R^8$ für Methyl steht, zu verwenden.

Die Aldehyde oder Ketone oder Aldehydderivate oder Ketonderivate oder Vinylether werden zur Synthese der 1,3-Dioxane der allgemeinen Formel V in einem äquivalenten Verhältnis oder in einem bis zu 10-fachen Überschuß mit den Triolen der allgemeinen Formel IV umgesetzt. Bevorzugt werden die Triole der Formel IV mit einem bis zwei Äquivalenten der Aldehyde bzw. Ketone bzw. deren angeführter Derivate umgesetzt.

Die Synthese dar 1,3-Dioxane der allgemeinen Formel V wird in Gegenwart von Säure durchgeführt. Geeignete Säuren sind Mineralsäuren wie Salzsäure oder Schwefelsäure, oder Chlorwasserstoff, oder organische Säuren wie Carbon- oder Sulfonsäuran wie z.B. Essigsäure, Monochloressigsäure, Dichloressigsäure, Trichloressigsäure, Trifluoressigsäure, Triphenylessigsäure, Methansulfonsäure, p-Toluolsulfonsäure oder saure Ionenaustauscherharze. Die Umsetzungen zu den 1,3-Dioxanen der allgemeinen Formel V können in Gegenwart von katalytischen Mengen bis zu 0,1 molaren Mengen der zugesetzten Säure durchgeführt werden. Bevorzugte Säuremengen sind katalytische Säuremengen.

Die Synthese der 1,3-Dioxane der allgemeinen Formel V kann bei Temperaturen von −30°C bis 100°C erfolgen. Bevorzugt sind Temperaturbereiche von 20°C bis 80°C.

Die auf diese Weise hergestellten Dioxane der allgemeinen Formel V werden nach Beendigung der Reaktion durch in der organischen Chemie übliche Verfahren wie Extraktion, Chromatographie oder Kristallisation gereinigt und isoliert.

Der nächste Schritt in der Synthese der erfindungsgemäßen Verbindungen der allgemeinen Formel I besteht in der Veretherung der C-3-Hydroxylgruppe in den Verbindungen der allgemeinen Formel V. Die Veretherungen werden bevorzugt mit geeigneten alpha-Halogencarbonsäuren durchgeführt in Gegenwart von starken Basen. Geeignete Basen sind beispielsweise Natriumhydroxid, Kaliumhydroxid, Bariumhydroxid oder Natriumhydrid. Geeignete alpha-Halogencarbonsäuren sind beispielsweise Chloressigsäure, 2-Chlorpropionsäure, 2-Chlorbuttersäure, 2-Chlorvaleriansäure, Bromessigsäure, 2-Brom-propionsäure oder 2-Bromvaleriansäure.

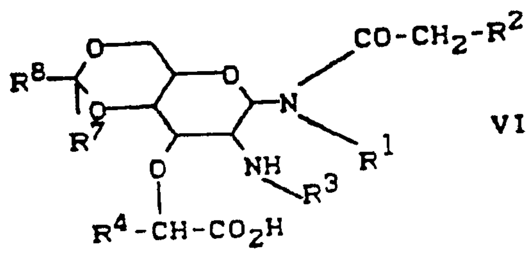

VI

Die Veretherung zur Herstellung der Verbindungen der allgemeinen Formel VI wird bevorzugt in einem organischen, inerten Lösungsmittel durchgeführt. Geeignete Lösungsmittel sich beispielsweise Ether wie Tetrahydrofuran, Dioxan, Ethylenglycoldimethylether, Ethylenglycoldiethylether, Diethylenglycoldimethylether, Amide wie N,N-Dimethylformamid, Hexomethylphosphoroäuretriamid oder Dimethylsulfoxid.

Die Veretherungsreaktion kann im Temperaturbereich von 20°C bis 100°C durchgeführt werden, bevorzugte Reaktionstemperaturen sind zwischen 20°C und 70°C. Die Reaktionszeiten betragen zwischen einigen Tagen und einigen Stunden, abhängig von Temperatur, Base und Halogencarbonsäure. Bei einer Reaktionstemperatur von 60°C, der Verwendung von Natriumhydrid als Base und bei Einsatz von 2-Chlorpropionsäure als Alkylierungsmittel beträgt die Reaktionszeit etwa 5 Stunden.

Die Hologencarbonsäure kann, bezogen auf den Alkohol, in einem Bereich von einem bis zu 10 Äquivalenten zugesetzt werden, bevorzugt wird ein etwa 2 bis 4-facher Überschuß an Halogencarbonsäure.

Die Umsetzung der Halogencarbonsäure mit dem Alkohol V verläuft, bezogen auf die Stereochemie am C-2 der Halogencarbonsäure, unter Walden-Umkehr. Wenn also eine optisch aktive 2-Halogencarbonsäure als Alkylierungsmittel eingesetzt wird, erhält man auch einen chiralen Milchsäureether. Bei Verwendung von L-2-Halogencarbonsäure ergibt die Alkylierung den D-Milchsäureether. Der Einsatz von D-2-Halogencarbonsäuren liefert L-Milchsäureether. Enantiomeren merenmischungen von 2-Halogencarbonsäuren ergeben Diastereomerenmischungen der betreffenden Milchsäureether VI.

Im Prinzip ähnliche Reaktionen zur Synthese von 3-O-Lactyl-glucosamiden, die am C-1 des Zuckers einen sauerstoffhaltigen Substituenten tragen (O-Glycoside), sind in der Literatur mehrfach beschrieben worden. Eine aktuelle Zusammenfassung dieser Aktivitäten ist bei A. Adam u.d E. Lederer, Med. Res. Rev. 4, 111 (1984) nachzulesen.

Der Unterschied zwischen den hier beschriebenen neuen Verbindungen der Formel I und den in der angegebenen Literaturstelle beschriebenen Arbeiten besteht u.a. darin, daß in den literaturbekannten Arbeiten ausschließlich O-Glycoside mit einem Milchsäurerest versehen werden. Im Unterschied zu den O-Glycosiden werden hier jedoch N-Glycoside in die 3-O-Milchsäurederivate übergeführt.

Die 1,3-Dioxane der allgemeinen Formel VI lassen sich unter geeigneten Bedingungen zu Dialkoholen der allgemeinen Formel VII spalten,

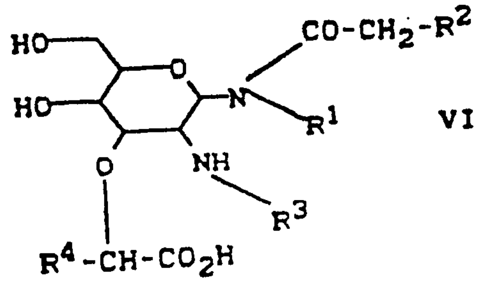

VII

Spaltungen von 1,3-Dioxanen, wie sie 4,6-O-Isopropyliden oder 4,6-O-Benzylidenverbindungen an Sacchariden darstellen, sind in der einschlägigen Literatur prinzipiell beschrieben, z.B. in "Methods in Carbohydrate Chemistry" Vol. 1, (1962), Seiten 69, 92, 111, 200, 201, 214, 245, 262, 284. In den vorliegenden Fällen zur Herstellung der Verbindungen der allgemeinen Formel VII erwies es sich als günstig, die 1,3-Dioxane der allgemeinen Formel VI in einem geeigneten Verdünnungsmittel mit schwachen, wässrigen Säuren zu behandeln. Geeignete Lösungsmittel waren hlaogenierte Kohlenwasserstoffe wie Dichlormethan, Chloroform, Ether wie 1,4-Dioxan oder Tetrahydrofuran, Alkohole wie Methanol, Ethanol, Propanol, Isopropanol oder Ester wie Essigsäureethylester, entweder allein oder im Gemisch miteinander. Im allgemeinen wurde Tetrahydrofuran oder 1,4-Dioxan bevorzugt verwendet.

Als verdünnte, wässrige Säuren können Mineralsäuren wie Salzsäure oder Schwefelsäure oder organische Säuren wie Essigsäure, Chloressgsäure, Dichloressigsäure, Trichloressigsäure oder Sulfonsäuren wie Methansulfonsäure oder Toluolsulfonsäure als Mischungen mit Wasser verwendet werden. Der Wassergehalt der zur Abspaltung geeigneten Gemische kann zwischen 1 und 99% betragen. Bei Verwendung von Essigsäure lag der bevorzugte Wassergehalt bei 40 bis 60%, im Falle von Trifluoressigsäure war es günstig, mit einem Wassergehalt von 5% zu arbeiten. Es ist auch möglich, die 1,3-Dioxanringe mit sauren Ionenaustauscherharzen zu spalten.

Die Reaktionstemperaturen für die Spaltung der 1,3-Dioxanringe in den Verbindungen der allgemeinen formel VI liegen im Bereich zwischen −20°C und 90°C, abhängig von der verwendeten Spaltmischung. Die bevorzugte Temperatur bei der Spaltreaktion mit wässriger Essigsäure lag im Bereich von 50°C bis 70°C, bei der Spaltung mit wässriger Trifluoressigsäure (95%) bei Raumtemperatur.

Die Diole der allgemeinen Formel VII ließen sich weiter zu den O-substituierten Derivaten der allgemeinen Formel VIII umsetzen.

Acylierungsreaktionen mit geeigneten Carbonsäurederivaten wie Anhydriden oder Carbonsäurechloriden in einem geeigneten Lösungsmittel, das an der Reaktion selbst nicht mit teilnimmt, in Gegenwart einer anorganischen oder organischen Base wie z.B. Pyridin oder Triethylamin ergaben die 4,6-Di-O-acylderivate der allgemeinen Formel I. Alkylierungen der Diole mit Alkylhalogeniden in Gegenwart von geeigneten Basen ergaben die 4,6-Di-O-alkylderivate der allgemeinen Formel I

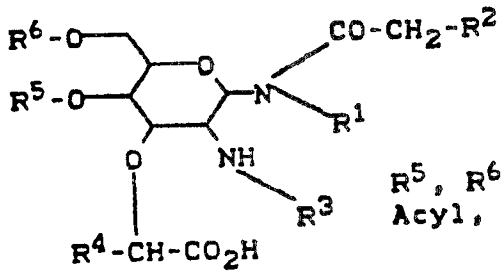

(I)

$R^5$, $R^6$ = Acyl, Alkyl

Das folgende Syntheseschema soll eine der bevorzugten Ausführungsformen der erfindungsgemäßen Darstellung von Verbindungen der Formel I beispielhaft erläutern.

N-Acetyl-D-glucosamin (a) wurde mit Dodecylamin (b) zum Glycosylamin umgesetzt, das mit dem gemischten Anhydrid aus Dodecansäure und Chlorammisensäureethylester selektiv N-acyliert wurde zum N-(2-Acetamido-2-desoxy-β-D-glucopyranosyl)-N-dodecyl-dodecansäureamid (d). Das Triol (d) wurde dann mit Benzaldebyddimethylacetal (e) in Gegenwart von Säure zur Benzylidenverbindung (f) umgesetzt. Die Umsetzung von (f) mit L-2-Chlorpropionsäure (g) lieferte unter Inversion un C-2 der Propionsäure das Muraminsäurederivat (h). Die Benzylidengruppe in (h) konnte dann durch Einwirkung mit verdünnten wässrigen Säuren abgespalten werden zur Verbindung (i), deren Diolgruppierung in Folgereaktionen sowohl acyliert als auch alkyliert werden konnte.

Die Erfindung betrifft generell auch die Salze der Verbindungen der allgemeinen Formel I mit irgendwelchen anderen salzbildenden Gruppen, in erster Linie pharmazeutisch verwendbare, nicht-toxische Salze, z.B. Alkali- oder Erdalkali- oder Ammaniumsalze.

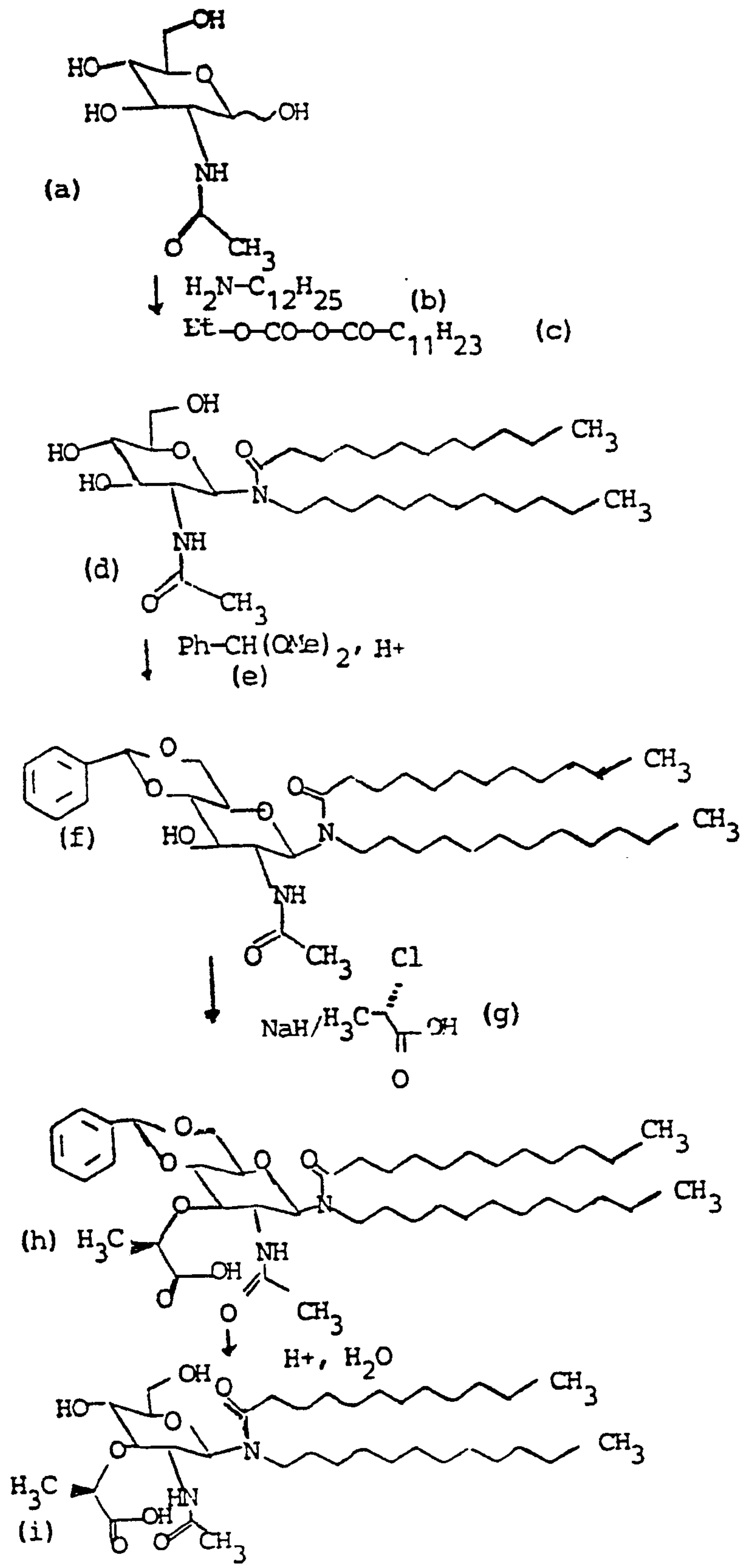


Die Erfindungsgemäßen Verbindungen weisen eine breite Abwehr-steigernde Wirkung auf.

Substanzen, die die körpereigene Abwehr (Immunsystem, Phagocytose) während einer Infektion stimulieren, sind sowohl für die Human- wie für die Veterinärmedizin von großem Interesse, da viele Infektionen trotz guter chemotherapeutischer Möglichkeiten ohne Unterstützung durch körpereigene Abwehrmechanismen persistieren. Dies kann zum erneuten Auftreten von Symptomen (Rezidiv) nach dem Überstehen der Ersterkrankung und damit zu chronisch rezidivierenden Krankheiten führen. Unter den durch Bakterien bedingten Erkrankungen sind es besonders Infektionen mit fakultativ intrazellulären Bakterien, die Probleme darstellen.

Ein Experimentalmodell für eine solche Erkrankung stellt die Infektion der Maus mit Salmonella typhimurium dar. Nach Inokulation der Mäuse mit diesen humanpathogenen Bakterien kommt es in Abhängigkeit von der Infektionsdosis zu einem subakut bis chronischen Krankheitsverlauf, bei dem die Tiere erst nach 4 bis 7 Tagen abzusterben beginnen. Während dieses Zeitraumes besteht die Möglichkeit, das Immunsystem durch Substanzen zu beeinflussen. Während der ersten zwei Wochen werden hohe Keimzahlen im Blut und in Leber und Milz infizierter Tiere gefunden. Die Keimzahlen nehmen danach allmählich ab, sind aber noch 8-12 Wochen nach der Inokulation nachweisbar. Bei den meisten anderen tierexperimentellen Infektionen, kommt es zu einem sehr schnellen Absterben der Tiere innerhalb von 1 bis 2 Tagen. Damit besteht keine Möglichkeit mehr, die Abwehr während der Infekfion zu stimulieren.

Es ist ferner bekannt, daß N-Acetyl-muramyl-L-alanyl-D-isoglutamin, die kleinste wirksame Komponente aus der Zellwand von Mykobakterien, die unspezifische Infektionsabwehr stimuliert (Robert Koch Stiftung e.V., Beiträge und Mitteilungen Vol. 5/1983, S. 31-38).

Ausserdem sind in der EP-A-0091645 N-glycosylierte Carbonsäureamid-Derivate beschrieben, die ebenfalls eine immunstimulierende Wirkung haben. Die in dieser Druckschrift offenbarten Verbindungen enthalten jedoch keinen 3-O-Lactylrest.

Es wurde nun überraschenderweise gefunden, daß auch die erfindungsgemäßen Verbindungen der allgemeinen Formel I die unspezifische Abwehr gegenüber Infekfionen steigern können. Dies wurde anhand der folgenden Versuche gefunden :

Die erfindungsgemäßen Verbindungen der allgemeinen Formel I wurden Mäusen entweder einmalig vor der Infekfion intraperitoneal oder an 2 aufeinanderfolgenden Tagen einmal täglich per os in unterschiedlichen Dosen verabfolgf, und zwar 1 Tag vor und am Tage der intraperitonealen Infekfion mit $2 \times 10^5$ Kolonien-bildenden Einheiten (KbE) von Salmonella typhimurium. Diese Infektionsdosis führt bei unbehandelten Tieren am 3. Tag zu einer hohen Keimzahl im Blut und in den Organen, insbesondere Leber und Milz. Die Tiere wurden in Makrolon-Käfigen unter konstanten Bedingungen (22° ± 2°C ; 55-65% relative Luftfeuchtigkeit) gehalfen und erhielten Sniff Versuchstierdiät.

In mehreren Versuchen kam es nach Behandlung der Tiere mit den erfindungsgemäßen Verbindungen der allgemeinen Formel I in Dosierungen von 1,10 oder 100 mg/kg Wirkstoff zu einer signifikanten Verminderung der Heimzahlen infizierter Mäuse im Blut im Vergleich zu Tieren, die nicht behandelt worden waren.

Diese Wirkungen wurden sowohl bei peroraler als auch bei parenteraler Gabe der Substanzen der allgemeinen Formel I gefunden. Sie führen bei oraler oder parenteraler Gabe zu einer deutlichen Verminderung der Bakterienzahlen im Blut und in der Leber und zwar nach intraperitonealer und intravenöser Infektion mit sog. intrazellulären Bakterien, d.h. Bakterien, die sich nach Aufnahme in die Makrophagenden wichtigsten Zellen der unspezifischen Abwehr – so lange weitervermehren, bis diese Zellen im Immunsystem aktiviert und damit in die Lage versetzt werden, die Bakterien intrazellulär abzutöten.

Dies soll beispielhaft für die Substanzen der Formel I anhand der Verbindung C2 verdeutlicht werden.

Tabelle

Wirkung von Verbindung C2 bei oraler Gabe 24 Stunden vor und am Tage der Infektion, jeweils 1 × pro Tag. Die Infektion erfolgte intraperitoneal mit S. typhimurium ($2 \times 10^5$ kolonienbildende Einheiten (KbE) pro Maus, entspr. $10 \times LD_{50}$)

| Dosis (mg/kg) | Keimzahlen drei Tage nach der Infektion (% der Kontrolle) | |
|---|---|---|
| | KbE/ml Blut | KbE/g Leber |
| 10 | 4** | 25* |
| 100 | 8 | 25* |
| Kontrolle | 100 % | 100 % |

Unterschiede zur Kontrolle

* p ˜ 0.05 Student's t-Test

** p ⟨ 0.01

Da die Substanzen der allgemeinen Formel I in vitro keinen direkten Effekt auf die Vermehrung von Salmonella typhimurium zeigten, ist anzunehmen, daß der Effekt der Substanzen auf einer Abwehrsteigerung des Wirtes gegenüber den Bakterien beruht. Dies führt zu einer Erhöhung der Überlebensrate infizierter Tiere und zu einem schnelleren Abklingen von Krankheitssymptomen.

Die pharmazeutischen Präparate der vorliegenden Erfindung sind vorzugsweise Tabletten oder Gelatinekapseln, welche die Wirkstoffe zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salze davon, wie Magnesium- oder Calcium-stearat, und/oder Polyethylenglykol, enthalten. Tabletten enthalten ebenfalls Bindemittel, z.B. Magnesium-aluminium-silikat, Stärken, wie Mais-, Weizen-, Reis- oder Pfeilwurzstärke, Gelatine, Traganth, Methylcellulose, Natriumcarboxymethylcellulose und/oder Polyvinylpynolidon, und wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmackstoffe und Süßmittel. Injizierbare Präparate sind vorzugsweise isotonische wäßrige Lösungen oder Suspensionen. Suppositorien, Salben oder Cremes sind in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten, Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragier- verfahren, hergestellt und enthalten von etwa 0,1 /. bis etwa 75% insbesondere von etwa 1%. bis 50% der genannten Aktivstoffe.

Die oral applizierten Präparate der vorliegenden Erfindung können auch mit einem gegen Magensaft beständigen Überzug versehen werden.

## Beispiele

A) Durch Umsetzung von 2-Acylamido-2-desoxy-D-hexopyranose (II) mit Amin $R^1$-$NH_2$ und anschließend mit dem gemischten Anhydrid aus Carbonsäure und Chlorameisensäureethylester gemäß DE-OS 3 213 650 wurden die folgenden Derivate der allgemeinen Formel IV hergestellt.

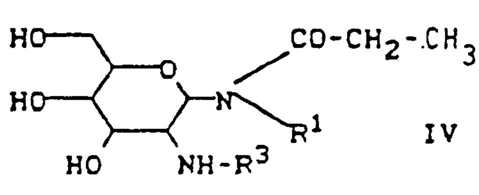

IV

| Nr. | $R^1$ | $R^2$ | $R^3$ | Zucker |
|---|---|---|---|---|
| A1 | -H | $-(CH_2)_{10}-CH_3$ | $-CO-CH_3$ | D-gluco |
| A2 | $-(CH_2)_{11}-CH_3$ | $-(CH_2)_{10}-CH_3$ | $-CO-CH_3$ | D-gluco |
| A3 | $-(CH_2)_{11}-CH_3$ | $-(CH_2)_{12}-CH_3$ | $-CO-CH_3$ | D-gluco |
| A4 | $-(CH_2)_{11}-CH_3$ | $-(CH_2)_{14}-CH_3$ | $-CO-CH_3$ | D-gluco |
| A5 | $-(CH_2)_{11}-CH_3$ | $-(CH_2)_{16}-CH_3$ | $-CO-CH_3$ | D-gluco |
| A6 | $-(CH_2)_{11}-CH_3$ | $-(CH_2)_{10}-CH_3$ | $-CO-CH_2-CH_3$ | " |
| A7 | $-(CH_2)_{11}-CH_3$ | $-(CH_2)_{10}-CH_3$ | $-CO-(CH_2)_2-CH_3$ | " |
| A8 | $-(CH_2)_{13}-CH_3$ | $-(CH_2)_{10}-CH_3$ | $-CO-CH_3$ | " |
| A9 | $-(CH_2)_{13}-CH_3$ | $-(CH_2)_{12}-CH_3$ | $-CO-CH_3$ | " |
| A10 | $-(CH_2)_{13}-CH_3$ | $-(CH_2)_{14}-CH_3$ | $-CO-CH_3$ | " |
| A11 | $-(CH_2)_{13}-CH_3$ | $-(CH_2)_{16}-CH_3$ | $-CO-CH_3$ | " |
| A12 | $-(CH_2)_{15}-CH_3$ | $-(CH_2)_{10}-CH_3$ | $-CO-CH_3$ | " |

| Nr. | $R^1$ | $R^2$ | $R^3$ | Zucker |
|---|---|---|---|---|
| A13 | $-(CH_2)_{15}-CH_3$ | $-(CH_2)_{12}-CH_3$ | $-CO-CH_3$ | D-gluco |
| A14 | $-(CH_2)_{15}-CH_3$ | $-(CH_2)_{14}-CH_3$ | $-CO-CH_3$ | " |
| A15 | $-(CH_2)_{15}-CH_3$ | $-(CH_2)_{16}-CH_3$ | $-CO-CH_3$ | " |
| A16 | $-(CH_2)_{17}-CH_3$ | $-(CH_2)_{10}-CH_3$ | $-CO-CH_3$ | " |
| A17 | $-(CH_2)_{17}-CH_3$ | $-(CH_2)_{12}-CH_3$ | $-CO-CH_3$ | " |
| A18 | $-(CH_2)_{17}-CH_3$ | $-(CH_2)_{14}-CH_3$ | $-CO-CH_3$ | " |
| A19 | $-(CH_2)_{17}-CH_3$ | $-(CH_2)_{16}-CH_3$ | $-CO-CH_3$ | " |
| A20 | $-(CH_2)_{11}-CH_3$ | $-(CH_2)_{16}-CH_3$ | $-CO-CH_3$ | " |
| A21 | $-(CH_2)_{13}-CH_3$ | $-(CH_2)_{16}-CH_3$ | $-CO-CH_3$ | D-galacto |
| A22 | $-(CH_2)_{15}-CH_3$ | $-(CH_2)_{16}-CH_3$ | $-CO-CH_3$ | " |
| A23 | $-(CH_2)_{17}-CH_3$ | $-(CH_2)_{16}-CH_3$ | $-CO-CH_3$ | " |

B) Allgemeines Verfahren zur Herstellung der 1,3-Dioxanderivate der Formel V. ($R^7$ = Phenyl, $R^8$ = H)

25 mmol des Triols der allgemeinen Formel IV wurden in 100 ml absolutem Tetrahydrofuran gelöst, mit 27,5 mmol Benzaldehyddimethylacetal und 10 mg p-Toluolsulfonsäure versetzt und mehrere Stunden auf 60°C erwärmt. Der Fortgang der Reaktion wurde dünnschichtchromatographisch auf Kieselgel verfolgt (Laufmittel : Toluol-Ethanol = 5 : 1). Nach Beendigung der Reaktion wurde die Säure mit basischem Ionenaustauscherharz neutralisiert. Das Harz wurde abfiltriert, Das Filtrat wurde im Vakuum eingedampft und zweimal nach Aufnahme in Toluol wieder zum Sirup eingedampft. Kristallisation aus Petrolether.

Ausbeuten ca. 75-95%.

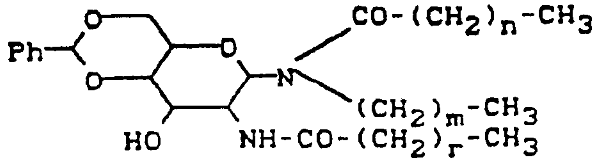

V ($R^7$=Phenyl, $R^8$=H)

| Nr. | Konfig a) | m | n | r | Rf b) | Schmp. | $[\alpha]_D$ c) |
|---|---|---|---|---|---|---|---|
| B1 | D-glc | d) | d) | 0 | | | |
| B2 | D-glc | 11 | 10 | 0 | 0,19 | 124° | -0,2° |
| B3 | D-glc | 11 | 12 | 0 | 0,20 | 119 | |
| B4 | D-glc | 11 | 14 | 0 | 0,19 | 120 | -0,2° |
| B5 | D-glc | 11 | 16 | 0 | 0,20 | 119 | +0,1° |
| B6 | D-glc | 11 | 10 | 1 | | | |
| B7 | D-glc | 11 | 10 | 2 | | | |
| B8 | D-glc | 13 | 10 | 0 | 0,18 | 126 | +0,8° |
| B9 | D-glc | 13 | 12 | 0 | 0,18 | 120 | -0,4° |
| B10 | D-glc | 13 | 14 | 0 | | | |
| B11 | D-glc | 13 | 16 | 0 | 0,19 | 119 | +0,5° |
| B12 | D-glc | 15 | 10 | 0 | | | |
| B13 | D-glc | 15 | 12 | 0 | | | |
| B14 | D-glc | 15 | 14 | 0 | | | |
| B15 | D-glc | 15 | 16 | 0 | | | |
| B16 | D-glc | 17 | 10 | 0 | 0,18 | 117 | 0,6° |
| B17 | D-glc | 17 | 12 | 0 | 0,20 | 112 | -0,4° |
| B18 | D-glc | 17 | 14 | 0 | | | |
| B19 | D-glc | 17 | 16 | 0 | 0,21 | 119 | -0,6° |
| B20 | D-gal | 11 | 16 | 0 | | | |
| B21 | D-gal | 13 | 16 | 0 | | | |
| B22 | D-gal | 15 | 16 | 0 | | | |
| B23 | D-gal | 17 | 16 | 0 | | | |

a) D-glc: D-Glucose; D-gal: D-Galactose

b) Laufmittel: Toluol-Aceton = 3:1

c) Lösungsmittel: Tetrahydrofuran

d) C-1-Substituent am Zucker: $-NH-CO-(CH_2)_{10}-CH_3$

C) Allgemeines Verfahren zur Herstellung der D-Milchsäureether der allgemeinen Formel VI.

30,0 mmol der Dioxanverbindung der allgemeinen Formel V wurden in 800 ml 1,4-Dioxan gelöst und nach Zugabe von 150 mmol Natriumhydrid auf 95°C erwärmt. Nach 5 min. wurde auf 60°C abgekühlt. Zu dem Ansatz wurden 110 mmol L-2-Chlorpropionsäure, gelöst in 50 ml 1,4-Dioxan, getropft. Die Reaktiontemperatur wurde mehrere Stunden auf 60°C gehalten. Der fortgang der Reaktion wurde dünnschichtchromatographisch auf Kieselgel verfolgt (Laufmittel : Toluol-Ethanol = 5 : 1).

Nach dem Ende der Reaktion wurde der Überschuß an Natriumhydrid durch vorsichtige Zugabe von 2-Propanol zersetzt, anschließend wurde der Ansatz auf Eiswasser gegeben und mit 1 N Salzsäure auf pH 2,5 eingestellt. Die wässrige Phase wurde mit 500 ml Ether extrahiert, die Etherphasen wurden 2 mal mit je 100 ml Wasser rückextrahiert. Die Etherphase wurde über Magnesiumsulfat getrocknet und zum Sirup eingedampft. Der erbaltene Rückstand wurde aus Methanol kristallisiert.
Ausbeuten ca. 70-90%.

Die folgenden Derivate wurden nach dieser Vorschrift hergestellt :

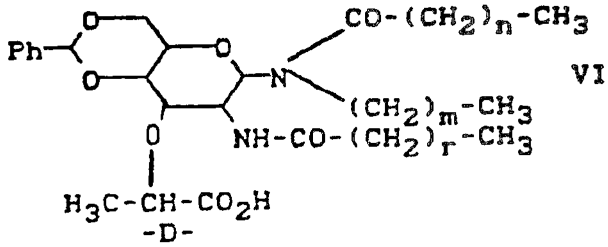

VI ($R^7$=Phenyl, $R^8$=H, $R^4$=$CH_3$)

| Nr. | Konfig[a)] | m | n | r | Rf[b)] | Schmp. | $[\alpha]_D$ [c)] |
|---|---|---|---|---|---|---|---|
| C1 | D-glc | d) | d) | 0 | | | |
| C2 | " | 11 | 10 | 0 | 0,36 | 130° | +5,5° |
| C3 | " | 11 | 12 | 0 | 0,36 | 117° | +5,2° |
| C4 | " | 11 | 14 | 0 | 0,37 | 130° | +5,4° |
| C5 | " | 11 | 16 | 0 | 0,41 | 127° | +5,6° |
| C6 | " | 11 | 10 | 1 | | | |
| C7 | " | 11 | 10 | 2 | | | |
| C8 | " | 13 | 10 | 0 | 0,35 | 143° | +5,4° |
| C9 | " | 13 | 12 | 0 | 0,36 | 130° | +5,5° |
| C10 | " | 13 | 14 | 0 | | | |
| C11 | " | 13 | 16 | 0 | 0,39 | 107° | +5,6° |
| C12 | " | 15 | 10 | 0 | | | |
| C13 | " | 15 | 12 | 0 | | | |
| C14 | " | 15 | 14 | 0 | | | |
| C15 | " | 15 | 16 | 0 | | | |
| C16 | " | 17 | 10 | 0 | 0,38 | 114° | +4,7° |
| C17 | " | 17 | 12 | 0 | 0,38 | 117° | +4,7° |
| C18 | " | 17 | 14 | 0 | | | |
| C19 | " | 17 | 16 | 0 | 0,40 | 108° | +4,9° |
| C20 | D-gal | 11 | 16 | 0 | | | |
| C21 | " | 13 | 16 | 0 | | | |
| C22 | " | 15 | 16 | 0 | | | |
| C23 | " | 17 | 16 | 0 | | | |

a) D-glc: D-Glucose; D-gal: D-Galactose

b) Laufmittel Toluol: Ethanol = 3:1

c) Lösungsmittel: Tetrahydrofuran

d) C-1-Substituent am Zucker: $-NH-CO-(CH_2)_{10}-CH_3$

Durch Einsatz anderer 2-Halogencarbonsäuren als L-2-Chlorpropionsäure wurden nach der oben genannten allgemeinen Vorschrift zur Herstellung der Verbindungen der Formel VI die folgenden Verbindungen hergestellt:

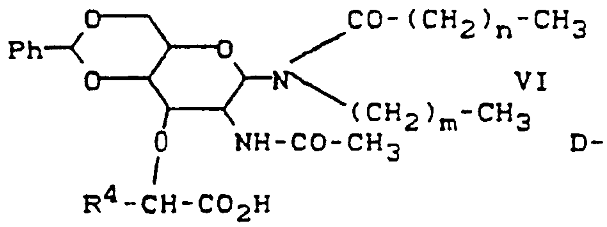

VI ($R^7$=Ph, $R^8$=H)

D-Glucose-Konfiguration

| Nr. | m | n | eingesetzte Säure | $R^4$ | Rf[a] |
|---|---|---|---|---|---|
| C24 | 11 | 10 | $Br-CH_2-CO_2H$ | H | 0,35 |
| C25 | 11 | 16 | $Br-CH_2-CO_2H$ | H | 0,37 |
| C26 | 13 | 16 | $Br-CH_2-CO_2H$ | H | |
| C27 | 17 | 16 | $Br-CH_2-CO_2H$ | H | 0,37 |
| C28 | 11 | 10 | $H_5C_2-CH(Br)-CO_2H$[b] | $C_2H_5$ | 0,37 |
| C29 | 17 | 10 | $H_5C_2-CH(Br)-CO_2H$ | $C_2H_5$ | 0,38 |

a) Laufmittel Toluol: Ethanol = 3:1

b) Enantiomerenmischung

D) Allgemeine Vorschriften zur Herstellung der Diole der allgemeinen Formel VII.

10 mmol der 1,3-Dioxanverbindung der allgemeinen Formel VI wurden in 150 ml Eisessig gelöst und auf 80°C erwärmt. Zu der Mischung wurden unter Rühren 20 ml Wasser zugetropft, die Reaktionsmischung wurde weiterhin auf 80°C gehalten. Der Fortgang der Reaktion wurde dünnschichchromatographisch auf Kieselgel verfolgt (Laufmittel Toluol-Ethanol-Eisessig = 10 : 2 : 0,15). Nach ca. 3 h wurde der Ansatz auf Raumtemperatur abgekühlt. Der danach ausgefallene farblose Feststoff wurde abgesaugt, mit Wasser gewaschen und getrocknet.

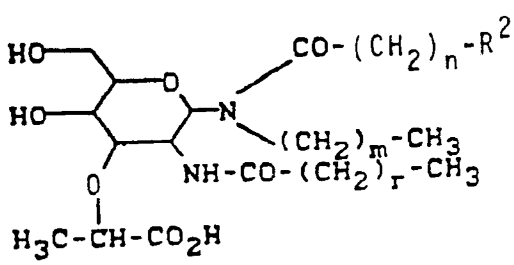

VI

| Nr. | Konfig.[a)] | m | n | r | Rf[b)] | [α][c)] |
|---|---|---|---|---|---|---|
| D1 | D-glc | d) | d) | 0 | | |
| D2 | " | 11 | 10 | 0 | 0,24 | +23,3° |
| D3 | " | 11 | 12 | 0 | 0,24 | +23,2° |
| D4 | " | 11 | 14 | 0 | 0,25 | +22,5° |
| D5 | " | 11 | 16 | 0 | 0,25 | +20,3° |
| D6 | " | 11 | 10 | 1 | | |
| D7 | " | 11 | 10 | 2 | | |
| D8 | " | 13 | 10 | 0 | 0,23 | +22,4° |
| D9 | " | 13 | 12 | 0 | 0,23 | +22,9° |
| D10 | " | 13 | 14 | 0 | | |
| D11 | " | 13 | 16 | 0 | 0,24 | +23,4° |
| D12 | " | 15 | 10 | 0 | | |
| D13 | " | 15 | 12 | 0 | | |
| D14 | " | 15 | 14 | 0 | | |
| D15 | " | 15 | 16 | 0 | | |
| D16 | " | 17 | 10 | 0 | 0,24 | +22,8° |
| D17 | " | 17 | 12 | 0 | 0,25 | +22,1° |
| D18 | " | 17 | 14 | 0 | | |
| D19 | " | 17 | 16 | 0 | 0,26 | +21,5° |
| D20 | D-gal | 11 | 16 | 0 | | |
| D21 | " | 13 | 16 | 0 | | |
| D22 | " | 15 | 16 | 0 | | |
| D23 | " | 17 | 16 | 0 | | |

a) D-glc: D-Glucose; D-gal: D.Galactose

b) Laufmittel: Toluol-Ethanol-Eisessig = 10:2:0,15

c) Lösungsmittel: Tetrahydrofuran

d) C-1-Substituent am Zucker: $-NH-CO(CH_2)_{10}-CH_3$

Unter gleichen Bedingungen wurden auch die Verbindungen C 24 bis C 29 in die Diole der Formel VI übergeführt.

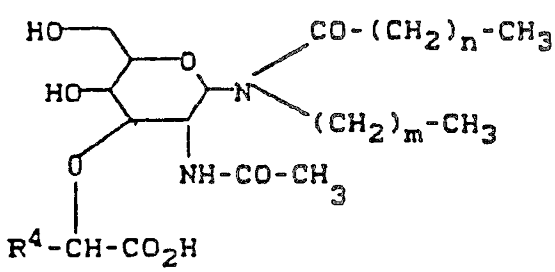

| Nr. | m | n | $R^4$ | Rf a) |
|---|---|---|---|---|
| D24 | 11 | 10 | H | 0,24 |
| D25 | 11 | 16 | H | 0,25 |
| D26 | 13 | 16 | H | 0,24 |
| D27 | 17 | 16 | H | 0,25 |
| D28 | 11 | 10 | $C_2H_5$ | 0,26 |
| D29 | 17 | 10 | $C_2H_5$ | 0,26 |

a) Laufmittel Toluol-Ethanol-Eisessig = 10:2:0,15

## Ansprüche

1. Verbindungen der allgemeinen Formel I

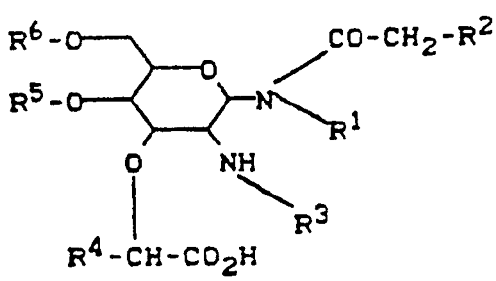

(I)

in der

$R^1$ Wasserstoff oder einen gesättigten oder einfach oder mehrfach ungesättigten Alkylrest mit einem bis zu 50 Kohlenstoffatomen,

$R^2$ Wasserstoff oder einen gesättigten oder einfach oder mehrfach ungesättigten Alkylrest mit einem bis zu 50 Kohlenstoffatomen,

$R^3$ Wasserstoff oder einen Acylrest mit bis zu 20 Kohlenstoffatomen,

$R^4$ Wasserstoff oder einen Alkylrest mit bis zu 4 Kohlenstoffatomen,

$R^5$ und $R^6$ unabhängig voneinander Wasserstoff oder einen Acylrest mit bis zu 10 Kohlenstoffatomen oder einen gegebenenfalls ungesättigten Alkylrest oder einen Aralkylrest mit bis zu 10 Kohlenstoffatomen,

$R^5$ und $R^6$ zusammen eine Gruppierung

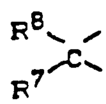

bedeuten,

wobei $R^7$ und $R^8$ unabhängig voneinander Wasserstoff oder Niederalkyl mit bis zu 5 Kohlenstoffatomen oder einen gegebenenfalls substituierten Arylrest mit bis zu 10-C-Atomen im aromatischen Teil bedeuten.

2. Verbindungen gemäß Anspruch 1, dadurch gekennzeichnet, daß

$R^1$ und $R^2$ für einen geradkettigen, gesättigten oder einfach oder mehrfach ungesättigten Alkylrest mit bis zu 22 Kohlenstoffatomen stehen,

$R^3$ Wasserstoff oder einen kurzkettigen Acylrest mit bis zu 5 Kohlenstoffatomen bedeutet,

$R^5$ und $R^6$ unabhängig voneinander für Wasserstoff oder einen kurzkettigen aliphatischen, gesättigten Acylsubstituenten mit bis zu 5 Kohlenstoffatomen oder einen aromatischen Acylsubstituenten wie Benzoyl oder p-Methoxybenzoyl oder für einen ungesättigten Acylrest mit bis zu 5 C-Atomen oder für Benzyl oder p-Methoxybenzyl stehen oder

$R^5$ und $R^6$ zusammen eine Benzyliden-, p-Methoxy-benzyliden- oder Isopropyliden-Gruppe bilden.

3. Verbindungen gemäß Anspruch 1 oder 2, dadurch gekennzeichnet, daß

$R^1$ und $R^2$, gegebenenfalls unabhängig voneinander, für geradkettige, gesättigte oder einfach ungesättigte Alkylreste mit 10 bis 20 Kohlenstoffatomen stehen,

$R^3$ für Formyl, Acetyl, Propionyl, Butyryl oder Valeryl steht und

$R^4$ Methyl, Ethyl, n-Propyl, i-Propyl oder Butyl bedeutet.

4. Verbindungen nach den Ansprüchen 1 bis 3, dadurch gekennzeichnet, daß $R^1$ und $R^2$ gleich oder verschieden sind und für Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Heneicosyl, Docosyl, Tricosyl, Tetracosyl, Hexacosyl, Octacosyl, Triacontyl, Vinyl, Allyl, But-2-enyl, But-3-enyl, Hex-2-enyl, Hex-3-enyl, Hex-4-enyl, Hex-5-enyl, Oct-2-enyl, Oct-4-enyl, Oct-6-enyl, Dec-2-enyl-, Dec-4-enyl, dec-6-enyl, Dec-8-enyl, Dodec-2-enyl, Dodec-4-enyl, Dodec-6-enyl, Dodec-8-enyl, Dodec-10-enyl, Tetradec-2-enyl, Tetradec-4-enyl, Tetradec-6-enyl, Tetradec-8-enyl, Tetradec-10-enyl, Tetradec-12-enyl, Hexadec-2-enyl, Hexadec-4-enyl, Hexadec-6-enyl, Hexadec-8-enyl, Hexadec-10-enyl, Hexadec-12-enyl, Hexadec-14-enyl, Octadec-2-enyl, Octadec-4-enyl, Octadec-6-enyl, Octadec-8-enyl, Octadec-10-enyl, Octadec-12-enyl, Octadec-14-enyl, Octadec-16-enyl, Heptadec-8,11-dienyl, Heptadec-8,11,14-trienyl stehen.

5. Verbindungen nach den Ansprüchen 1 bis 4, dadurch gekennzeichnet, daß der Zuckerrest in den allgemeinen Formel I ein 2-Amino-2-desoxy-D-glucose- oder 2-Amino-2-desoxy-D-galactose-Rest ist.

6. Verfahren zur Herstellung von Verbindungen der allgemeinen Formel I

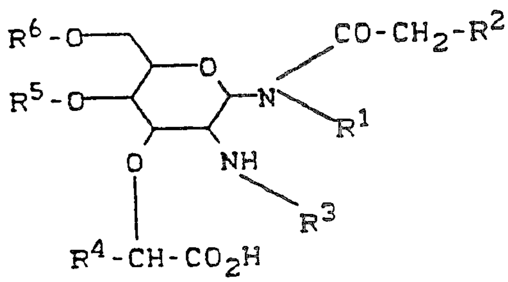

(I)

in der

$R^1$ Wasserstoff oder einen gesättigten oder einfach oder mehrfach ungesättigten Alkylrest mit einem bis zu 50 Kohlenstoffatomen.

$R^2$ Wasserstoff oder einen gesättigten oder einfach oder mehrfach ungesättigten Alkylrest mit einem bis zu 50 Kohlenstoffatomen.

$R^3$ Wasserstoff oder einen Acylrest mit bis zu 20 Kohlenstoffatomen.

$R^4$ Wasserstoff oder einen Alkylrest mit bis zu 4 Kohlenstoffatomen.

$R^5$ und $R^6$ unabhängig voneinander Wasserstoff oder einen Acylrest mit bis zu 10 Kohlenstoffatomen oder einen gegebenenfalls ungesättigten Alkylrest oder einen Aralkylrest mit bis zu 10 Kohlenstoffatomen.

$R^6$ Wasserstoff oder einen Acylrest mit bis zu 10 Kohlenstoffatomen oden einen Alkylrest mit bis zu 10 Kohlenstoffatomen oder

$R^5$ und $R^6$ zusammen eine Gruppierung

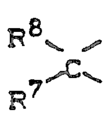

bedeuten,

wobei $R^7$ und $R^8$ unabhängig voneinander Wasserstoff oder Niederalkyl mit bis zu 5 Kohlenstoffatomen oder einen gegebenenfalls substituierten Arylrest mit bis zu 10-C-Atomen im aromatischen Teil bedeuten, dadurch gekennzeichnet, daß man an der Aminogruppe acylierte Derivate von 2-Amino-2-desoxy-hexosen der allge-

meinen Formel II

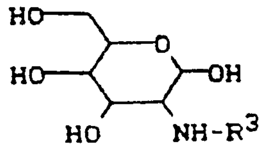

II

mit der oben genannten Bedeutung von $R^3$ mit Aminoverbindungen $R^1$-$NH_2$ mit der oben genannten Bedeutung für $R^1$ zu einem Glycosylamin der allgemeinen Formel III

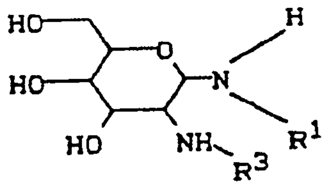

III

mit den oben genannten Bedeutungen für $R^1$ und $R^3$ umsetzt und das Glycosylamin der allgemeinen Formel III nach dessen Isolierung mit einem aktivierten Carbonsäurederivat selektiv N-acyliert zum Glycosylamid der allgemeinen Formel IV,

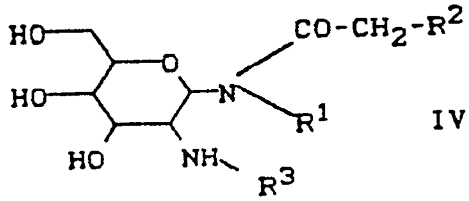

IV

mit den oben angegebenen Bedeutungen von $R^1$, $R^2$ und $R^3$, die Verbindungen der allgemeinen Formel IV mit einem Aldehyd oder einem Keton oder einem Aldehydderivat oder einem Ketonderivat zu den Verbindungen der allgemeinen Formel V

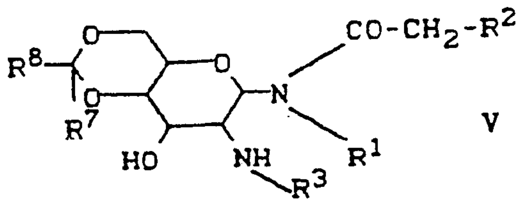

V

umsetzt, die auf diese Weise hergestellten Dioxane der allgemeinen Formel V nach Beendigung der Reaktion durch in der organischen Chemie übliche Verfahren wie Extraktion, Chromatographie oder Kristallisation reinigt oder isoliert, die C-3-Hydroxylgruppe in den Verbindungen der allgemeinen Formel V verethert zu Verbindungen der Formel VI

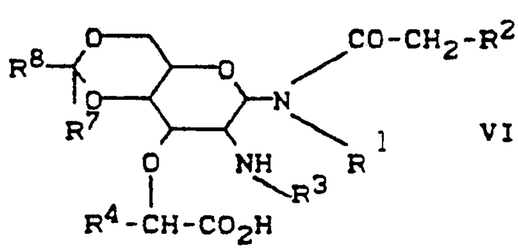

VI

mit den oben angegebenen Restedefinitionen, die 1,3-Dioxane der allgemeinen Formel VI zu Dialkoholen der

allgemeinen Formel VII spaltet und letztere zu den Verbindungen der Formel I acyliert oder alkyliert.

7. Verfahren nach Anspruch 6, dadurch gekennzeichnet, daß man die Verbindungen der Formel IV mit Aceton, Methylethylketon, Methylpropylketon, Methylbutylketon, Methylpentylketon, Diethylketon, Ethylpropylketon oder Dipropylketone oder mit Acetaldehyddimethylacetal, Acetaldehyddiethylacetal, Propionaldehyddimethylacetal, Propionaldehyddiethylacetal, Benzaldehyddimethylacetal, Benzaldehyddiethylacetal, p-Tolualdehyddimethylacetal, p-Tolualdehyddiethylacetal oder p-Anisaldehyddimethylacetal, p-Anisaldehyddiethylacetal, 2,2-Dimethoxypropan, 2,2-Diethoxypropan, 2,2-Dimethoxybutan, 2,2-Diethoxybutan, 2,2-Dimethoxypentan, 2,2-Diethoxypentan, 3,3-Dimethoxypentan, 3,3-Diethoxypentan, Acetophenon-dimethylketal, Acetophenondiethyketal oder 2-Propen-2-yl-methylether zu Verbindungen der allgemeinen Formel V umsetzt.

8. Verfahren nach Anspruch 7, dadurch gekennzeichnet, daß man die Umacetalisierungsreaktion in Tetrahydrofuran, 1,4-Dioxan, Dimethylformamid, Dichlormethan, Chloroform, Ethylenglykoldimethylether oder in Mischungen dieser Lösungsmittel oder in einem Überschuß des als Lösungsmittel und Reagenz fungierenden Aldehyds oder Ketons oder Aldehydderivates oder Ketonderivates durchführt.

9. Verfahren nach Anspruch 8, dadurch gekennzeichnet, daß man die Umsetzung der Verbindungen der Formel IV zu den Verbindungen der Formel V mit einem 1 – bis 10-fachen Überschuß an Aldehyd oder Keton oder deren Derivaten in Gegenwart von katalytischen bis 0,1 molaren Mengen eine Säure aus der Gruppe bestehend aus Essigsäure, Monochloressissäure, Dichloressigsäure, Trichloressigsäure, Trifluoressigsäure, Triphenylessigsäure, Methansulfonsäure und p-Toluolsulfonsäure oder in Gegenwart saurer Ionenaustauscher durchführt.

10. Verfahren nach den Ansprüchen 7 bis 9, dadurch gekennzeichnet, daß man die Reaktion bei Temperaturen von –30°C bis 100°C, insbesondere von 20°C bis 80°C durchführt.

11. Verfahren nach Anspruch 6, dadurch gekennzeichnet, daß man die Veretherungsreaktion zu den Verbindungen der Formel VI bei Temperaturen von 20°C bis 100°C mit 1 bis 10 Äquivalenten einer α-Halogencarbonsäure in Gegenwart einer starken Base durchführt.

12. Verfahren nach Anspruch 6, dadurch gekennzeichnet, daß die 1,3-Dioxane der allgemeinen Formel VI in Dichlormethan, Chloroform, 1,4-Trioxan, Tetrahydrofuran, Methanol, Ethanol, Propanol, Isopropanol oder Essigsäureethylester oder in Gemischen dieser Lösungsmittel in Gegenwart wäßriger, schwacher Säuren zu Verbindungen der Formel VII gespalten werden.

13. Verfahren nach Anspruch 6, dadurch gekennzeichnet, daß man die Verbindungen der Formel VII zu den 4,6-Tri-O-acylderivaten oder 4,6-Tri-O-alkylderivaten der Formel I mit Carbonsäurederivaten oder Alkylhalogeniden in Gegenwart von Basen umsetzt.

14. Verbindungen der allgemeinen Formel I

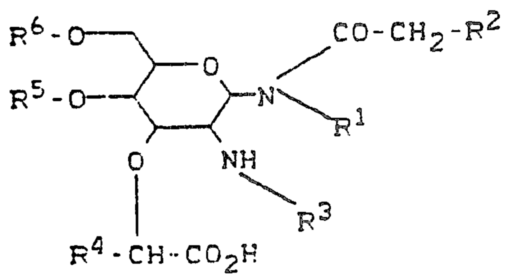

(I)

in der

$R^1$ Wasserstoff oder einen gesättigten oder einfach oder mehrfach ungesättigten Alkylrest mit einem bis zu 50 Kohlenstoffatomen.

$R^2$ Wasserstoff oder einen gesättigten oder einfach oder mehrfach ungesättigten Alkylrest mit einem bis zu 50 Kohlenstoffatomen.

$R^3$ Wasserstoff oder einen Acylrest mit bis zu 20 Kohlenstoffatomen.

$R^4$ Wasserstoff oder einen Alkylrest mit bis zu 4 Kohlenstoffatomen.

$R^5$ und $R^6$ unabhängig voneinander Wasserstoff oder einen Acylrest mit bis zu 10 Kohlenstoffatomen oder einen gegebenenfalls ungesättigten Alkylrest oder einen Aralkylrest mit bis zu 10 Kohlenstoffatomen.

$R^5$ und $R^6$ zusammen eine Gruppierung

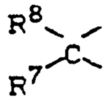

bedeuten,
wobei $R^7$ und $R^8$ unabhängig voneinander Wasserstoff oder Niederalkyl mit bis zu 5 Kohlenstoffatomen oder einen gegebenenfalls substituierten Arylrest mit bis zu 10 C-Atomen im aromatischen Teil bedeuten, zur Verwendung in einem Verfahren zur therapeutischen Behandlung des menschlichen oder tierischen Körpers.

15. Arzneimittel, enthaltend Verbindungen der allgemeinen Formel I

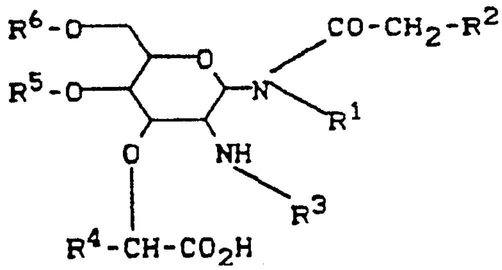

(I)

in der

$R^1$ Wasserstoff oder einen gesättigten oder einfach oder mehrfach ungesättigten Alkylrest mit einem bis zu 50 Kohlenstoffatomen,

$R^2$ Wasserstoff oder einen gesättigten oder einfach oder mehrfach ungesättigten Alkylrest mit einem bis zu 50 Kohlenstoffatomen,

$R^3$ Wasserstoff oder einen Acylrest mit bis zu 20 Kohlenstoffatomen,

$R^4$ Wasserstoff oder einen Alkylrest mit bis zu 4 Kohlenstoffatomen,

$R^5$ und $R^6$ unabhängig voneinander Wasserstoff oder einen Acylrest mit bis zu 10 Kohlenstoffatomen oder einen gegebenenfalls ungesättigten Alkylrest oder einen Aralkylrest mit bis zu 10 Kohlenstoffatomen,

$R^5$ und $R^6$ zusammen eine Gruppierung

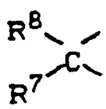

bedeuten,
wobei $R^7$ und $R^8$ unabhängig voneinander für Wasserstoff oder Niederalkyl mit bis zu 5 Kohlenstoffatomen oder einen gegebenenfalls substituierten Arylrest mit bis zu 10 C-Atomen im aromatischen teil bedeuten.

16. Verwendung von Verbindungen der allgemeinen Formel I

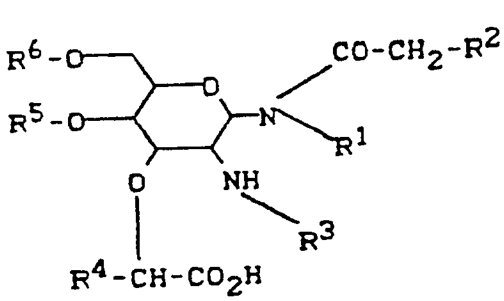

(I)

in der

$R^1$ Wasserstoff oder einen gesättigten oder einfach oder mehrfach ungesättigten Alkylrest mit einem bis zu 50 Kohlenstoffatomen,

$R^2$ Wasserstoff oder einen gesättigten oder einfach oder mehrfach ungesättigten Alkylrest mit einem bis zu 50 Kohlenstoffatomen,

$R^3$ Wasserstoff oder einen Acylrest mit bis zu 20 Kohlenstoffatomen,

$R^4$ Wasserstoff oder einen Alkylrest mit bis zu 4 Kohlenstoffatomen,

$R^5$ und $R^6$ unabhängig voneinander Wasserstoff oder einen Acylrest mit bis zu 10 Kohlenstoffatomen oder einen gegebenenfalls ungesättigten Alkylrest oder einen Aralkylrest mit bis zu 10 Kohlenstoffatomen,

$R^5$ und $R^6$ zusammen eine Gruppierung

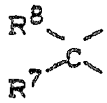

bedeuten,
wobei $R^7$ und $R^8$ unabhängig voneinander für Wasserstoff oder Niederalkyl mit bis zu 5 Kohlenstoffatomen oder einen gegebenenfalls substituierten Arylrest mit bis zu 10 C-Atomen im aromatischen Teil bedeuten zur Herstellung von Arzneimitteln.

## Claims

1. Compounds of the general formula I

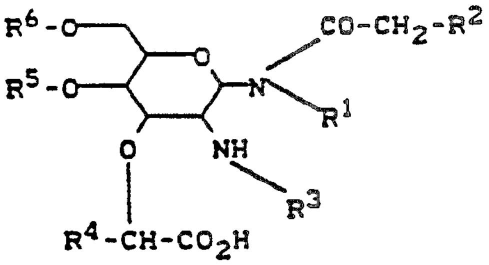

(I)

in which

$R^1$ denotes hydrogen or a saturated or mono- or polyunsaturated alkyl radical with one to 50 carbon atoms,

$R^2$ denotes hydrogen or a saturated or mono- or polyunsaturated alkyl radical with one to 50 carbon atoms,

$R^3$ denotes hydrogen or an acyl radical with up to 20 carbon atoms,

$R^4$ denotes hydrogen or an alkyl radical with up to 4 carbon atoms and

$R^5$ and $R^6$ independently of one another denote hydrogen or an acyl radical with up to 10 carbon atoms or an optionally unsaturated alkyl radical or an aralkyl radical with up to 10 carbon atoms, or

$R^5$ and $R^6$ together denote a grouping

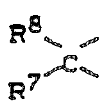

wherein

$R^7$ and $R^8$ independently of one another denote hydrogen or lower alkyl with up to 5 carbon atoms or an optionally substituted aryl radical with up to 10 C atoms in the aromatic moiety.

2. Compounds according to Claim 1, characterised in that

$R^1$ and $R^2$ represent a straight-chain, saturated or mono- or polyunsaturated alkyl radical with up to 22 carbon atoms and

$R^3$ denotes hydrogen or a short-chain acyl radical with up to 5 carbon atoms,

$R^5$ and $R^6$ independently of one another represent hydrogen or a short-chain aliphatic, saturated acyl substituent with up to 5 carbon atoms or an aromatic acyl substituent, such as benzoyl or p-methoxybenzoyl, or represent an unsaturated acyl radical with up to 5 C atoms or represent benzyl or p-methoxybenzyl, or

$R^5$ and $R^6$ together form a benzylidene, p-methoxybenzylidene or isopropylidene group.

3. Compounds according to Claim 1 or 2, characterised in that

$R^1$ and $R^2$ independently of one another represent straight-chain, saturated or monounsaturated alkyl radicals with 10 to 20 carbon atoms,

$R^3$ represents formyl, acetyl, propionyl, butyryl or valeryl and

$R^4$ denotes methyl, ethyl, n-propyl, i-propyl or butyl.

4. Compounds according to Claims 1 to 3, characterised in that $R^1$ and $R^2$ are identical or different and represent methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl, tricosyl, tetracosyl, hexacosyl, octacosyl, triacontyl, vinyl, allyl, but-2-enyl, but-3-enyl, hex-2-enyl, hex-3-enyl, hex-4-enyl, hex-5-enyl, oct-2-enyl, oct-4-enyl, oct-6-enyl, dec-2-enyl, dec-4-enyl, dec-6-enyl, dec-8-enyl, dodec-2-enyl, dodec-4-enyl, dodec-6-enyl, dodec-8-enyl, dodec-10-enyl, tetra-dec-2-enyl, tetradec-4-enyl, tetradec-6-enyl, tetradec-8-enyl, tetradec-10-enyl, tetradec-12-enyl, hexadec-2-enyl, hexadec-4-enyl, hexadec-6-enyl, hexadec-8-enyl, hexadec-10-enyl, hexadec-12-enyl, hexadec-14-enyl, octadec-2-enyl, octadec-4-enyl, octadec-6-enyl, octadec-8-enyl, octadec-10-enyl, octadec-12-enyl, octadec-14-enyl, octadec-16-enyl, heptadec-8,11-dienyl or heptadec-8,11,14-trienyl.

5. Compounds according to Claims 1 to 4, characterised in that the sugar radical in the general formula I is a 2-amino-2-deoxy-D-glucose or 2-amino-2-deoxy-D-galactose radical.

6. Process for the preparation of compounds of the general formula I

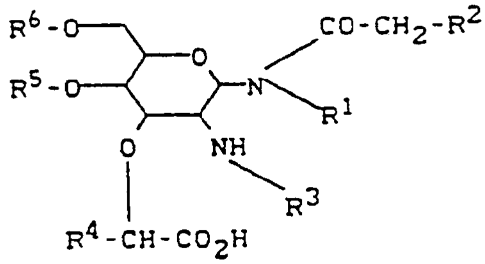

(I)

in which

$R^1$ denotes hydrogen or a saturated or mono- or polyunsaturated alkyl radical with one to 50 carbon atoms,

$R^2$ denotes hydrogen or a saturated or mono- or polyunsaturated alkyl radical with one to 50 carbon atoms,

$R^3$ denotes hydrogen or an acyl radical with up to 20 carbon atoms,

$R^4$ denotes hydrogen or an alkyl radical with up to 4 carbon atoms and

$R^5$ and $R^6$ independently of one another denote hydrogen or an acyl radical with up to 10 carbon atoms or an optionally unsaturated alkyl radical or an aralkyl radical with up to 10 carbon atoms,

$R^6$ denotes hydrogen or an acyl radical with up to 10 carbon atoms or an alkyl radical with up to 10 carbon atoms, or

$R^5$ and $R^6$ together denote a grouping

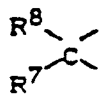

wherein

$R^7$ and $R^8$ independently of one another denote hydrogen or lower alkyl with up to 5 carbon atoms or an optionally substituted aryl radical with up to 10 C atoms in the aromatic moiety,

characterised in that derivatives of 2-amino-2-deoxyhexoses acylated on the amino group, of the general formula II

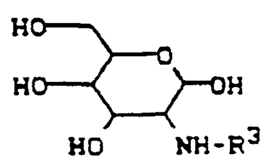

II

with the abovementioned meaning of $R^3$, are reacted with amino compounds $R^1$-$NH_2$, with the abovementioned meaning for $R^1$, to give a glycosylamine of the general formula III

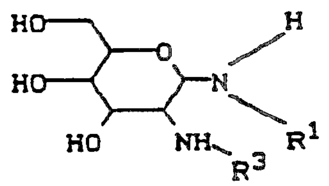

III

with the abovementioned meanings for $R^1$ and $R^3$ and the glycosylamine of the general formula III, after being isolated, is then selectively N-acylated with an activated carboxylic acid derivative to give the glycosylamide of the general formula IV

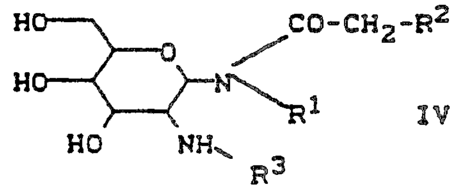

IV

with the abovementioned meanings of $R^1$, $R^2$ and $R^3$, the compounds of the general formula IV are reacted with an aldehyde or a ketone or an aldehyde derivative or a ketone derivative to give the compounds of the general formula V

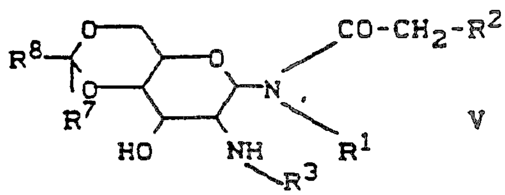

V

and after the reaction has ended, the dioxanes prepared in this manner, of the general formula V, are purified or isolated by processes customary in organic chemistry, such as extraction, chromatography or crystallisation, the C-3 hydroxyl group in the compounds of the general formula V is etherified to give compounds of the formula VI

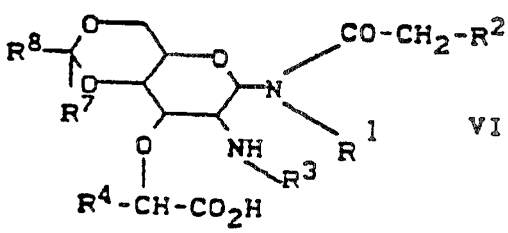

VI

with the abovementioned radical definitions, the 1,3-dioxanes of the general formula VI are split to give dialcohols of the general formula VII and the latter are acylated or alkylated to give the compounds of the formula I.

7. Process according to Claim 6, characterised in that the compounds of the formula IV are reacted with acetone, methyl ethyl ketone, methyl propyl ketone, methyl butyl ketone, methyl pentyl ketone, diethyl ketone, ethyl propyl ketone or dipropyl ketones or with acetaldehyde dimethyl acetal, acetaldehyde diethyl acetal, propionaldehyde dimethyl acetal, propionaldehyde diethyl acetal, benzaldehyde dimethyl acetal, benzaldehyde diethyl acetal, p-tolualdehyde dimethyl acetal, p-tolualdehyde diethyl acetal or p-anisaldehyde dimethyl acetal, p-anisaldehyde diethyl acetal, 2,2-dimethoxypropane, 2,2-diethoxypropane, 2,2-dimethoxybutane, 2,2-diethoxybutane, 2,2-dimethoxypentane, 2,2-diethoxypentane, 3,3-dimethoxypentane, 3,3-diethoxypentane, acetophenone dimethyl ketal, acetophenone diethyl ketal or 2-propen-2-yl methyl ether, to give compounds of the general formula V.

8. Process according to Claim 7, characterised in that the trans-acetalisation reaction is carried out in tetrahydrofuran, 1,4-dioxane, dimethylformamide, methylene chloride, chloroform or ethylene glycol dimethyl ether, or in mixtures of these solvents, or in an excess of the aldehyde or ketone or aldehyde derivative or ketone

derivative functioning as the solvent and reagent.

9. Process according to Claim 8, characterised in that the reaction of the compounds of the formula IV to give the compounds of the formula V is carried out with a 1- to 10-fold excess of aldehyde or ketone or derivatives thereof in the presence of catalytic to 0.1 molar amounts of an acid from the group consisting of acetic acid, monochloroacetic acid, dichloroacetic acid, trichloroacetic acid, trifluoroacetic acid, triphenylacetic acid, methanesulphonic acid and p-toluenesulphonic acid, or in the presence of acid ion exchangers.

10. Process according to Claims 7 to 9, characterised in that the reaction is carried out at temperatures from −30°C to 100°C, in particular from 20°C to 80°C.

11. Process according to Claim 6, characterised in that the etherification reaction to give the compounds of the formula VI is carried out at temperatures from 20°C to 100°C with 1 to 10 equivalents of an α-halogenocarboxylic acid in the presence of a strong base.

12. Process according to Claim 6, characterised in that the 1,3-dioxanes of the general formula VI are split in methylene chloride, chloroform, 1,4-trioxane, tetrahydrofuran, methanol, ethanol, propanol, isopropanol or ethyl acetate or in mixtures of these solvents in the presence of aqueous, weak acids, to give compounds of the formula VII.

13. Process according to Claim 6, characterised in that the compounds of the formula VII are reacted with carboxylic acid derivatives or alkyl halides in the presence of bases to give the 4,6-tri-O-acyl derivatives or 4,6-tri-O-alkyl derivatives of the formula I.

14. Compounds of the general formula I

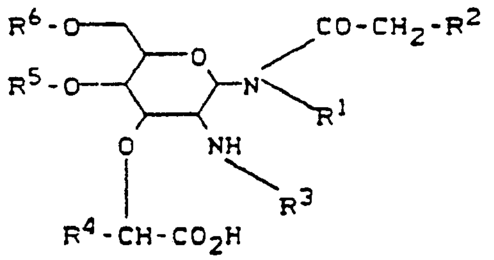

(I)

in which

$R^1$ denotes hydrogen or a saturated or mono- or polyunsaturated alkyl radical with one to 50 carbon atoms,

$R^2$ denotes hydrogen or a saturated or mono- or polyunsaturated alkyl radical with one to 50 carbon atoms,

$R^3$ denotes hydrogen or an acyl radical with up to 20 carbon atoms,

$R^4$ denotes hydrogen or an alkyl radical with up to 4 carbon atoms and

$R^5$ and $R^6$ independently of one another denote hydrogen or an acyl radical with up to 10 carbon atoms or an optionally unsaturated alkyl radical or an aralkyl radical with up to 10 carbon atoms, or

$R^5$ and $R^6$ together denote a grouping

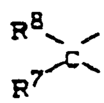

wherein

$R^7$ and $R^8$ independently of one another denote hydrogen or lower alkyl with up to 5 carbon atoms or an optionally substituted aryl radical with up to 10 C atoms in the aromatic moiety,

for use in a method for the therapeutic treatment of the human or animal body.

15. Medicaments containing compounds of the general formula I

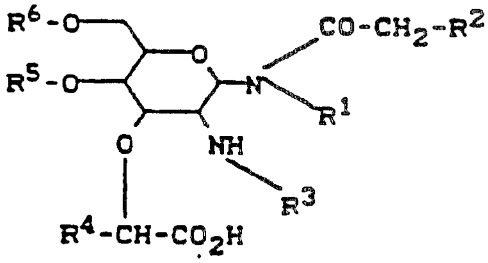

(I)

in which

$R^1$ denotes hydrogen or a saturated or mono- or polyunsaturated alkyl radical with one to 50 carbon atoms,

$R^2$ denotes hydrogen or a saturated or mono- or polyunsaturated alkyl radical with one to 50 carbon atoms,

$R^3$ denotes hydrogen or an acyl radical with up to 20 carbon atoms,

$R^4$ denotes hydrogen or an alkyl radical with up to 4 carbon atoms and

$R^5$ and $R^6$ independently of one another denote hydrogen or an acyl radical with up to 10 carbon atoms or an optionally unsaturated alkyl radical or an aralkyl radical with up to 10 carbon atoms, or

$R^5$ and $R^6$ together denote a grouping

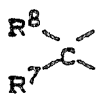

wherein

$R^7$ and $R^8$ independently of one another denote hydrogen or lower alkyl with up to 5 carbon atoms or an optionally substituted aryl radical with up to 10 C atoms in the aromatic moiety.

16. Use of compounds of the general formula I

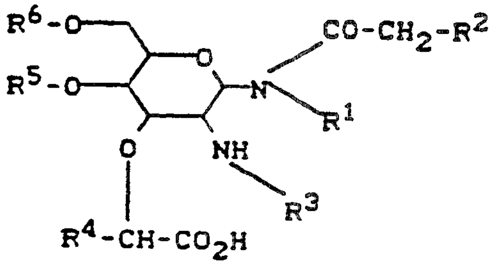

(I)

in which

$R^1$ denotes hydrogen or a saturated or mono- or polyunsaturated alkyl radical with one to 50 carbon atoms,

$R^2$ denotes hydrogen or a saturated or mono- or polyunsaturated alkyl radical with one to 50 carbon atoms,

$R^3$ denotes hydrogen or an acyl radical with up to 20 carbon atoms,

$R^4$ denotes hydrogen or an alkyl radical with up to 4 carbon atoms and

$R^5$ and $R^6$ independently of one another denote hydrogen or an acyl radical with up to 10 carbon atoms or an optionally unsaturated alkyl radical or an aralkyl radical with up to 10 carbon atoms, or

$R^5$ and $R^6$ together denote a grouping

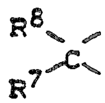

wherein

$R^7$ and $R^8$ independently of one another denote hydrogen or lower alkyl with up to 5 carbon atoms or an optionally substituted aryl radical with up to 10 C atoms in the aromatic moiety, for the preparation of medicaments.

## Revendications

1. Composés de formule I

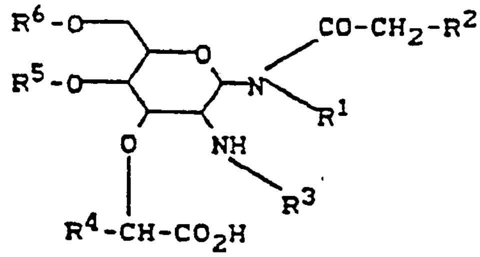

(I)

dans laquelle

$R^1$ représente l'hydrogène ou un reste alkyle saturé ou à une ou plusieurs insaturations, ayant jusqu'à 50 atomes de carbone,

$R^2$ représente l'hydrogène ou un reste alkyle saturé ou à une ou plusieurs insaturations ayant jusqu'à 50 atomes de carbone,

$R^3$ est l'hydrogène ou un reste acyle ayant jusqu'à 20 atomes de carbone,

$R^4$ est l'hydrogène ou un reste alkyle ayant jusqu'à 4 atomes de carbone,

$R^5$ et $R^6$ représentent, indépendamment l'un de l'autre, l'hydrogène ou un reste acyle ayant jusqu'à 10 atomes de carbone ou un reste alkyle éventuellement non saturé ou un reste aralkyle ayant jusqu'à 10 atomes de carbone,

$R^5$ et $R^6$ forment conjointement un groupement

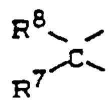

$R^7$ et $R^8$ désignant indépendamment l'un de l'autre l'hydrogène ou un groupe alkyle inférieur ayant jusqu'à 5 atomes de carbone ou un reste aryle éventuellement substitué ayant jusqu'à 10 atomes de carbone dans la partie aromatique.

2. Composés suivant la revendication 1, caractérisés en ce que

$R^1$ et $R^2$ représentent un reste alkyle à chaîne droite, saturé ou à une ou plusieurs insaturations, ayant jusqu'à 22 atomes de carbone,

$R^3$ représente l'hydrogène ou un reste acyle à chaîne courte ayant jusqu'à 5 atomes de carbone,

$R^5$ et $R^6$ représentent, indépendamment l'un de l'autre, l'hydrogène ou un substituant acyle aliphatique saturé à chaîne courte ayant jusqu'à 5 atomes de carbone ou un substituant acyle aromatique tel que benzoyle ou p-méthoxybenzoyle ou un reste acyle non saturé ayant jusqu'à 5 atomes de carbone ou un reste benzyle ou p-méthoxybenzyle, ou bien

$R^5$ et $R^6$ forment conjointement un groupe benzylidène, p-méthoxybenzylidène ou isopropylidène.

3. Composés suivant la revendication 1 ou 2, caractérisés en ce que

$R^1$ et $R^2$ représentent, le cas échéant, indépendamment l'un de l'autre, des restes alkyle à chaîne droite, saturés ou à mono-insaturation, de 10 à 20 atomes de carbone,

$R^3$ est un groupe formyle, acétyle, propionyle, butyryle ou valéryle et

$R^4$ est un groupe méthyle, éthyle, n-propyle, isopropyle ou butyle.

4. Composés suivant les revendications 1 à 3, caractérisés en ce que $R^1$ et $R^2$ sont identiques ou différents et représentent un groupe méthyle, éthyle, propyle, butyle, pentyle, hexyle, heptyle, octyle, nonyle, décyle, undécyle, dodécyle, tridécyle, tétradécyle, pentadécyle, hexadécyle, heptadécyle, octadécyle, nonadécyle, eicosyle, héneicosyle, docosyle, tricosyle, tétracosyle, hexacosyle, octacosyle, triacontyle, vinyle, allyle, but-2-ényle, but-3-ényle, hex-2-ényle, hex-3-ényle, hex-4-ényle, hex-5-ényle, oct-2-ényle, oct-4-ényle, oct-6-ényle, déc-2-ényle, déc-4-ényle, déc-6-ényle, déc-8-ényle, dodéc-2-ényle, dodéc-4-ényle, dodéc-6-ényle, dodéc-8-ényle, dodéc-10-ényle, tétradéc-2-ényle, tétradéc-4-ényle, tétradéc-6-ényle, tetradéc-8-ényle, tetradéc-10-ényle, tétradéc-12-ényle, hexadéc-2-ényle, hexadéc-4-ényle, hexadéc-6-ényle, hexadéc-8-ényle, hexadéc-10-ényle, hexadéc-12-ényle, hexadéc-14-ényle, octadéc-2-ényle, octadéc-4-ényle, octadéc-6-ényle, octadéc-8-ényle, octadéc-10-ényle, octadéc-12-ényle, octadéc-14-ényle, octadéc-16-ényle, heptadéc-8,11-diényle, heptadéc-8,11,14-triényle.

5. Composés suivant les revendications 1 à 4, caractérisés en ce que le reste sucre dans les formules générales I est un reste de 2-amino-2-désoxy-D-glucose ou de 2-amino-2-désoxy-D-galactose.

6. Procédé de préparation de composés de formule générale I

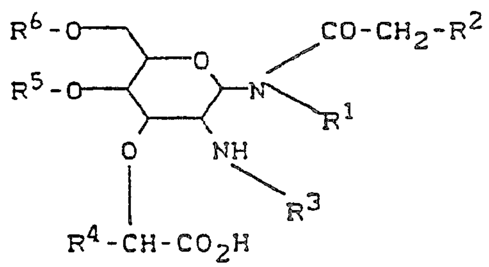

(I)

dans laquelle

$R^1$ représente l'hydrogène ou un reste alkyle saturé ou à une seule ou plusieurs insaturations ayant jusqu'à 50 atomes de carbone,

$R^2$ est l'hydrogène ou un reste alkyle saturé ou à une ou plusieurs insaturations ayant jusqu'à 50 atomes de carbone,

$R^3$ est l'hydrogène ou un reste acyle ayant jusqu'à 20 atomes de carbone,

$R^4$ est l'hydrogène ou un reste alkyle ayant jusqu'à 4 atomes de carbone,

$R^5$ et $R^6$ représentent, indépendamment l'un de l'autre, l'hydrogène ou un reste acyle ayant jusqu'à 10 atomes de carbone ou un reste alkyle éventuellement non saturé ou un reste aralkyle ayant jusqu'à 10 atomes de carbone,

$R^6$ est l'hydrogène ou un reste acyle ayant jusqu'à 10 atomes de carbone ou un reste alkyle ayant jusqu'à 10 atomes de carbone, ou bien

$R^5$ et $R^6$ forment conjointement un groupement

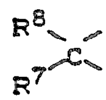

$R^7$ et $R^8$ représentant indépendamment l'un de l'autre l'hydrogène ou un groupe alkyle inférieur ayant jusqu'à 5 atomes de carbone ou un reste aryle éventuellement substitué ayant jusqu'à 10 atomes de carbone dans la partie aromatique, caractérisé en ce qu'on fait réagir des dérivés, acylés sur le groupe amino, de 2-amino-2-désoxy-hexoses de formule générale II

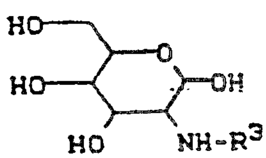

II

ayant la définition indiquée ci-dessus pour $R^3$ avec des composés aminés $R^1$-$NH_2$ avec la définition mentionnée ci-dessus pour $R^1$, pour former une glycosylamine de formule générale III

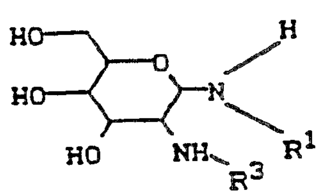

III

avec les définitions indiquées ci-dessus pour $R^1$ et $R^3$ et on acyle sur l'atome d'azote la glycosylamine de formule générale III après son isolement avec un dérivé activé d'acide carboxylique pour former le glycosylamide de formule générale IV,

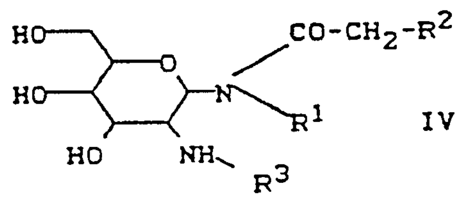

IV

avec les définitions indiquées ci-dessus pour $R^1$, $R^2$ et $R^3$, on fait réagir les composés de formule générale IV avec un aldéhyde ou une cétone ou avec un dérivé d'aldéhyde ou un dérivé de cétone pour former les composés de formule générale V

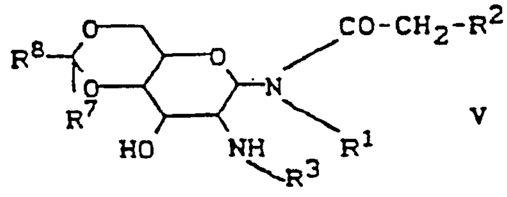

V

on purifie ou on isole les dioxannes ainsi préparés de formule générale V une fois terminée la réaction par des procédés d'emploi classique en chimie organique tels qu'extraction, chromatographie ou cristallisation, on éthérifie le groupe hydroxyle en position C-3 dans les composés de formule générale V en composés de formule VI

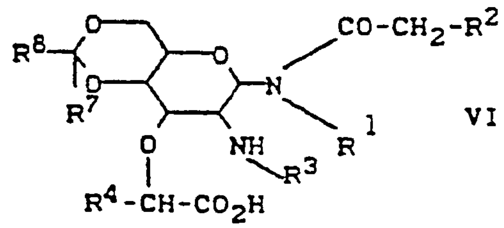

VI

avec les définitions indiquées ci-dessus, on scinde les 1,3-dioxannes de formule générale VI en dialcools de formule générale VII et on acyle ou alkyle ces derniers en les composés de formule I.

7. Procédé suivant la revendication 6, caractérisé en ce qu'on fait réagir les composés de formule IV avec l'acétone, la méthyléthylcétone, la méthylpropylcétone, la méthylbutylcétone, la méthylpentylcétone, la diéthylcétone, l'éthylpropylcétone ou des dipropylcétones ou avec l'acétaldéhyde-diméthylacétal, l'acétaldéhyde-diéthylacétal, le propionaldéhyde-diméthylacétal, le propionaldéhyde-diéthylacétal, le benzaldéhyde-diméthylacétal, le benzaldéhyde-diéthylacétal, le p-tolualdéhyde-diméthylacétal, le p-tolualdéhyde-diéthylacétal ou le p-anisaldéhyde-diméthylacétal, le p-anisaldéhyde-diéthylacétal, le 2,2-diméthoxypropane, le 2,2-diéthoxypropane, le 2,2-diméthoxybutane, le 2,2-diéthoxybutane, le 2,2-diméthoxypentane, le 2,2-diéthoxypentane, le 3,3-diméthoxypentane, le 3,3-diéthoxypentane, l'acétophénonediméthylcétal, l'acétophénonediéthylcétal ou l'éther de 2-propène-2-yl-méthyle pour obtenir des composés de formule générale V.

8. Procédé suivant la revendication 7, caractérisé en ce que la réaction de transacétalisation est conduite dans le tétrahydrofuranne, le 1,4-dioxanne, le diméthylformamide, le dichlorométhane, le chloroforme, l'éther diméthylique d'éthylèneglycol ou dans des mélanges de ces solvants ou dans un excès de l'aldéhyde ou de la cétone ou du dérivé d'acétaldéhyde ou du dérivé de cétone fonctionnant comme solvant et comme réactif.

9. Procédé suivant la revendication 8, caractérisé en ce que la réaction des composés de formule IV pour former des composés de formule V est conduite avec un excès d'un facteur 1 à 10 d'aldéhyde ou de cétone ou de leurs dérivés en présence de quantités allant de catalytiques à décimolaires d'un acide du groupe constitué de l'acide acétique, de l'acide monochloracétique, de l'acide dichloracétique, de l'acide trichloracétique, de l'acide trifluoracétique, de l'acide triphénylacétique, de l'acide méthanesulfonique et de l'acide p-toluènefulsonique ou en présence d'échangeurs ioniques acides.

10. Procédé suivant les revendications 7 à 9, caractérisé en ce qu'on conduit la réaction à des températures de –30°C à 100°C, notamment de 20°C à 80°C.

11. Procédé suivant la revendication 6, caractérisé en ce qu'on conduit la réaction d'éthérification pour former les composés de formule VI à des températures de 20°C à 100°C avec 1 à 10 équivalents d'un acide α-halogénocarboxylique en présence d'une base forte.

12. Procédé suivant la revendication 6, caractérisé en ce que les 1,3-dioxannes de formule générale VI sont scindées en composés de formule VII dans le dichlorométhane, le chloroforme, le 1,4-trioxanne, le tétrahydrofuranne, le méthanol, l'éthanol, le propanol, l'isopropanol ou l'ester éthylique d'acide acétique ou dans des mélanges de ces solvants en présence d'acides faibles aqueux.

13. Procédé suivant la revendication 6, caractérisé en ce qu'on fait réagir les composés de formule VII pour former les dérivés 4,6-tri-O-acyliques ou 4,6-tri-O-alkyliques de formule I avec des dérivés d'acides carboxyliques ou des halogénures d'alkyle en présence de bases.

14. Composés de formule générale I

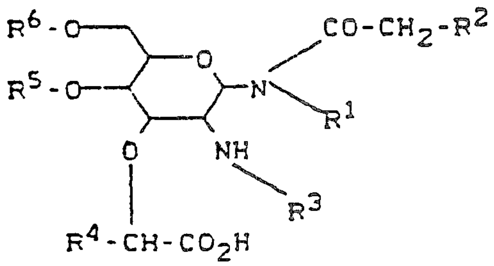

(I)

dans laquelle

$R^1$ représente l'hydrogène ou un reste alkyle saturé ou à une ou plusieurs insaturations ayant jusqu'à 50 atomes de carbone.

$R^2$ représente l'hydrogène ou un reste alkyle saturé ou à une ou plusieurs insaturations ayant jusqu'à 50 atomes de carbone.

$R^3$ représente l'hydrogène ou un reste acyle ayant jusqu'à 20 atomes de carbone.

$R^4$ représente l'hydrogène ou un reste alkyle ayant jusqu'à 4 atomes de carbone.

$R^5$ et $R^6$ représentent, indépendamment l'un de l'autre, l'hydrogène ou un reste acyle ayant jusqu'à 10 atomes de carbone ou un reste alkyle éventuellement insaturé ou un reste aralkyle ayant jusqu'à 10 atomes de carbone.

$R^5$ et $R^6$ forment conjointement un groupement

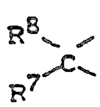

où $R^7$ et $R^8$ représentent, indépendamment l'un de l'autre, l'hydrogène ou un groupe alkyle inférieur ayant jusqu'à 5 atomes de carbone ou un reste aryle éventuellement susbtitué ayant jusqu'à 10 atomes de carbone dans la partie aromatique, destinés à être utilisés dans un procédé pour le traitement thérapeutique du corps humain ou animal.

15. Médicaments contenant des composés de formule générale I

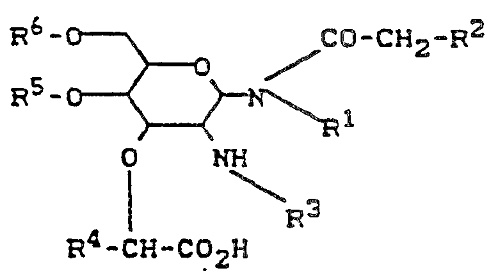

(I)

dans laquelle

$R^1$ représente l'hydrogène ou un reste alkyle saturé ou à une ou plusieurs insaturations ayant jusqu'à 50 atomes de carbone.

$R^2$ représente l'hydrogène ou un reste alkyle saturé ou à une ou plusieurs insaturations ayant jusqu'à 50 atomes de carbone.

$R^3$ représente l'hydrogène ou un reste acyle ayant jusqu'à 20 atomes de carbone.

$R^4$ représente l'hydrogène ou un reste alkyle ayant jusqu'à 4 atomes de carbone.

$R^5$ et $R^6$ représentent, indépendamment l'un de l'autre, l'hydrogène ou un reste acyle ayant jusqu'à 10 atomes de carbone ou un reste alkyle éventuellement insaturé ou un reste aralkyle ayant jusqu'à 10 atomes de carbone.

$R^5$ et $R^6$ forment conjointement un groupement

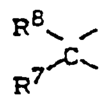

où $R^7$ et $R^8$ représentent, indépendamment l'un de l'autre, l'hydrogène ou un groupe alkyle inférieur ayant jusqu'à 5 atomes de carbone ou un reste aryle éventuellement susbtitué ayant jusqu'à 10 atomes de carbone dans la partie aromatique.

16. Utilisation de composés de formule

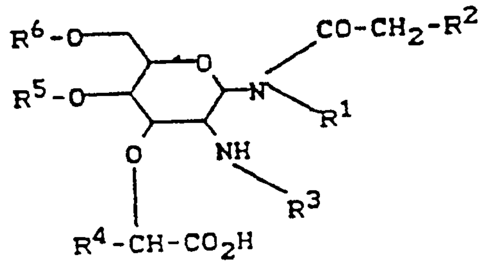

(I)

dans laquelle

$R^1$ représente l'hydrogène ou un reste alkyle saturé ou à une ou plusieurs insaturations ayant jusqu'à 50 atomes de carbone.

$R^2$ représente l'hydrogène ou un reste alkyle saturé ou à une ou plusieurs insaturations ayant jusqu'à 50 atomes de carbone.

$R^3$ représente l'hydrogène ou un reste acyle ayant jusqu'à 20 atomes de carbone.

$R^4$ représente l'hydrogène ou un reste alkyle ayant jusqu'à 4 atomes de carbone.

$R^5$ et $R^6$ représentent, indépendamment l'un de l'autre, l'hydrogène ou un reste acyle ayant jusqu'à 10 atomes de carbone ou un reste alkyle éventuellement insaturé ou un reste aralkyle ayant jusqu'à 10 atomes de carbone.

$R^5$ et $R^6$ forment conjointement un groupement

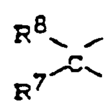

où $R^7$ et $R^8$ représentent, indépendamment l'un de l'autre, l'hydrogène ou un groupe alkyle inférieur ayant jusqu'a 5 atomes de carbone ou un reste aryle éventuellement substitué ayant jusqu'à 10 atomes de carbone dans la partie aromatique,
dans un procédé pour la préparation de médicaments.